# FASCICULE DE BREVET EUROPEEN

(11) **EP 2 649 084 B1**
(45) Date de publication et mention de la délivrance du brevet: **22.02.2017**
(21) Numéro de dépôt: 11811061.8
(22) Date de dépôt: 09.12.2011
(51) Int. Cl.: C07F 9/6584, A61P 35/00, A61P 37/06, A61K 31/675

(54) **NOUVEAUX DERIVES D'OXAZAPHOSPHORINES PRE-ACTIVEES, UTILISATION ET METHODE DE PREPARATION**
NEUE, VORAKTIVIERTE OXAZAPHOSPHORINDERIVATE, VERWENDUNG UND VERFAHREN ZU IHRER HERSTELLUNG
NOVEL DERIVATIVES OF OXAZAPHOSPHORINES THAT ARE PRE-ACTIVATED, USE AND METHOD OF PREPARATION

(30) Priorité: 10.12.2010 FR 1060350
(43) Date de publication de la demande: 16.10.2013
(73) Titulaire: Institut Gustave Roussy, 94805 Villejuif Cédex (FR)
(72) Inventeur: PACI, Angelo, F-92190 Meudon (FR); MARTENS, Thierry, F-94510 La Queue En Brie (FR); RIVARD, Michaël, F-94000 Creteil (FR); COUVREUR, Patrick, F-91140 Villebon Sur Yvette (FR); DESMAËL, Didier, F-94260 Fresnes (FR); CARON, Joachim, F-91940 Les Ulis (FR)
(74) Mandataire: Gallois, Valérie
(86) Numéro de dépôt international: PCT/FR2011/052914
(87) Numéro de publication internationale: WO 2012/076824

(56) Documents cités:
- GB-A- 2 095 256
- HIRANO T ET AL: "Synthesis of activated cyclophosphamide derivatives bearing functional groups", TETRAHEDRON LETTERS 1979 GB, vol. NO. 10, 1979, pages 883-886, XP002632109, ISSN: 0040-4039
- ZON G ET AL: "NMR spectroscopic studies of intermediary metabolites of cyclophosphamide. A comprehensive kinetic analysis of the interconversion of cis- and trans-4-hydroxyphosphamide with aldophosphamide and the concomitant partitioning of aldophosphamide between Irreversible fragmentation and reversible conjuga", JOURNAL OF MEDICINAL CHEMISTRY, AMERICAN CHEMICAL SOCIETY, WASHINGTON, US, vol. 27, no. 4, 1 janvier 1984 (1984-01-01), pages 466-485, XP003014079, ISSN: 0022-2623, DOI: DOI:10.1021/JM00370A008
- QIU RUOLUN ET AL: "ABCC2-mediated biliary transport of 4-glutathionylcyclophosphamide and its contribution to elimination of 4-hydroxycyclophosphamide in rat.", JOURNAL OF PHARMACOLOGY AND EXPERIMENTAL THERAPEUTICS, vol. 308, no. 3, mars 2004 (2004-03), pages 1204-1212, XP002632110, ISSN: 0022-3565
- REDDY L HARIVARDHAN ET AL: "Novel approaches to deliver gemcitabine to cancers", CURRENT PHARMACEUTICAL DESIGN, vol. 14, no. 11, avril 2008 (2008-04), pages 1124-1137, XP002632111, ISSN: 1381-6128

## Description

La présente invention se rapporte au domaine de la médecine, en particulier de l'oncologie. Elle concerne de nouveaux dérivés de médicaments oxazaphosphorines pré-activées.

Les médicaments de la classe des oxazaphosphorines sont des agents anticancéreux ou immunosuppresseur (CPM) alkylants utilisés dans le traitement du cancer (par exemple, les lymphomes et leucémies, les sarcomes, le cancer solide, notamment du poumon, du sein, de la prostate ou des ovaires). L'ifosfamide (IFM) et le cyclophosphamide (CPM) sont des prodrogues dont l'activité cytotoxique est associée à une activation métabolique cytochrome P450-dépendante introduisant un groupe hydroxyle en position 4. La toxicité des oxazaphosphorines (ifosfamide et cyclophosphamide) est d'ordre urologique et correspond à une accumulation d'acroléine, métabolite généré après libération de la moutarde alkylante. Par ailleurs, il existe également une toxicité d'ordre neurologique et néphrologique résultant de la présence du chloroacétaldéhyde, un métabolite issu de l'oxydation des chaînes latérales de la molécule via l'action des cytochromes P450. La toxicité due à l'acroléine peut être diminuée par une co-administration de mercaptoéthanesulfonate de sodium.

Une voie de synthèse par électrochimie de dérivés méthoxy en position 4 a été décrite (Paci et al, 2001, Bioorg Med Chem Lett, 11, 1347-1349). Ces dérivés sont considérés comme étant des analogues pré-activés puisque ceux-ci exhibent une activité cytotoxique comparable à celle du métabolite oxydé et beaucoup plus élevée que les produits initiaux non-oxydés. En effet, ils permettent la libération des moutardes alkylantes sans intervention des cytochromes P450.

Par ailleurs, d'autres dérivés d'oxazaphosphorines dans le but de diminuer leur toxicité ont été développés. Notamment, l'oxydation toxicogène des chaînes latérales méthylés de ces dérivés est supprimée ou limitée tout en conservant leur pouvoir alkylant. Pour revue, on peut consulter Giraud et al (2010, Expert Opin Drug Metab Toxicol, 6, 919-938). A titre d'illustration, on peut également citer deux dérivés du cyclophosphamide, à savoir le glufosfamide, et le mafosfamide, ainsi que le C7,C9-dimethyl-ifosfamide.

La demande de brevet GB2095256 décrit des acides oxazaphosphorin-4-thio-alkanesulphoniques, en particulier des dérivés du cyclophosphamide. Hirano et al. (Tetrahedron Letters, 10, 883-886) décrit également des dérivés du cyclophosphamide substitués en position 4 obtenus par réaction du 4-hydropéroxycyclophosphamide avec un mercaptoalkane.

GB2095256 et Hirano et al. ne décrivent aucun dérivé de l'ifosfamide substitué en position 4.

Zon et al. (J. Med. Chem., 1984, 27(4), 466-485) et Qiu et al. (J. Pharma. Exp. Therap., 2004, 308(3), 1204-12) concernent les voies de métabolisation et les métabolites du cyclophosphamide.Reddy et al. (Current Pharm. Design, 2008, 14(11), 1124-37) est un article de revue concernant la vectorisation de la gemcitabine. Reddy et al. décrit notamment des conjugués de la gemcitabine.

Des dérivés d'oxazaphosphorines, et en particulier des dérivés de l'ifosfamide, présentant une activité cytotoxique plus puissante ou présentant une action plus ciblés seraient très utiles, permettant ainsi de réduire la dose nécessaire et de diminuer ainsi les effets secondaires toxiques.

Les inventeurs ont mis au point une méthode permettant de préparer des dérivés d'oxazaphosphorines pré-activées et présentant un radical de vectorisation ou de formulation en position 4.

Un radical de vectorisation pourra permettre de cibler le tissu cible du médicament (les tissus cancéreux ou cellules cancéreuses) et par conséquent de diminuer les doses à administrer tout en conservant une bonne efficacité thérapeutique.

Un radical de formulation pourra permettre de protéger l'entité active d'une dégradation avant qu'il atteigne le tissu cible et/ou de moduler son activité via la modulation de la vitesse de libération de la moutarde alkylante, notamment en stabilisant la molécule. En effet, les oxazaphosphorines sont des médicaments qui se dégradent rapidement. En résumé, ce radical permet de modifier les propriétés physico-chimiques, pharmacocinétiques ou pharmacodynamiques des oxazaphosphorines.

L'invention concerne un composé de formule (I) tel que défini dans la revendication 1, à savoir : 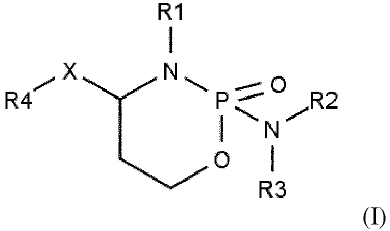 dans laquelle
- X est O ou S ;
- R2 est H ;
- R1 et R3 sont indépendamment -(CH₂)₂-Cl ou -CH(CH₃)-CH₂-Cl; et,
- R4 est de formule R5(Y)ₐ dans laquelle :
   - Y représente un espaceur, de préférence choisi parmi le groupe constitué par -(CH₂)ₘ-, -CONH(CH₂)ₘ-, -NHCO(CH₂)ₘ-, COO(CH₂)ₘ- et -OCO(CH₂)ₘ- avec m est un entier allant de 1 à 10 ;
   - a est 1 ; et
   - R₅ est un groupe hydrocarboné de 3 à 30 atomes de carbone, linéaire ou ramifié.
ou un sel pharmaceutiquement acceptable de celui-ci.

L'invention a également pour objet une méthode de préparation d'un composé de formule (I) telle que définie dans la revendication 16.

Il est décrit une méthode de préparation d'un dérivé d'oxazaphosphorines pré-activées de formule (I), 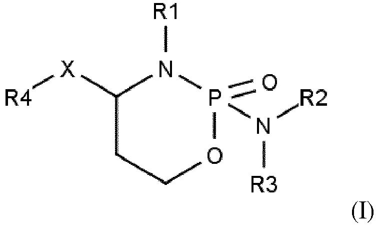 dans laquelle
X est O ou S ;
R1 et R2 sont, indépendamment, H, -CH(CH₃)-CH₂-Cl ou -(CH₂)₂-Cl, à condition qu'au moins un des deux soit -CH(CH₃)-CH₂-Cl ou -(CH₂)₂-Cl;
R3 est -CH(CH₃)-CH₂-Cl ou -(CH₂)₂-Cl ; et,
R4 est un radical de formulation ou de vectorisation comprenant au moins trois carbones ;
comprenant
- la fourniture d'un composé de formule (II) 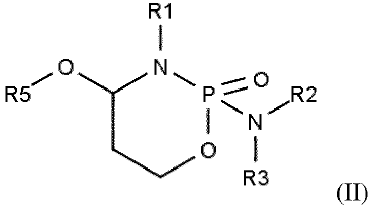 dans laquelle R1, R2 et R3 sont tels que définis dans la formule (I), et R5 est un méthyle ou un éthyle ; et,
- la réaction du composé de formule (II) avec un alcool ou thiol de formule (III) R4-XH, dans laquelle X et R4 sont tels que définis dans la formule (I), en présence d'un acide de Lewis.

Par « radical de formulation » est entendu un radical permettant de stabiliser l'oxazaphosphorine et notamment de diminuer la vitesse de libération de la moutarde alkylante. Notamment, la demi-vie de l'oxazaphosphorine pré-activée peut être augmentée par augmentation du nombre de carbone du radical de formulation. En outre, ce radical peut permettre ou améliorer la stabilité de l'oxazaphosphorine pour une administration parentérale et notamment intraveineuse. Dans un mode de réalisation tout particulièrement préféré, ce radical permet la formation de nanoparticules, de micelles ou de liposomes.

Par « radical de vectorisation » est entendu un radical permettant un ciblage de la tumeur, c'est-à-dire un adressage plus spécifique de l'oxazaphosphorine vers les tumeurs ou les cellules cancéreuses pour augmenter la spécificité d'action et diminuer les effets secondaires.

Facultativement, le radical comprend au moins 4, 5, 6, 7 ou 8 carbones.

La réaction chimique peut être décrite avec plus de détail dans le schéma suivant.

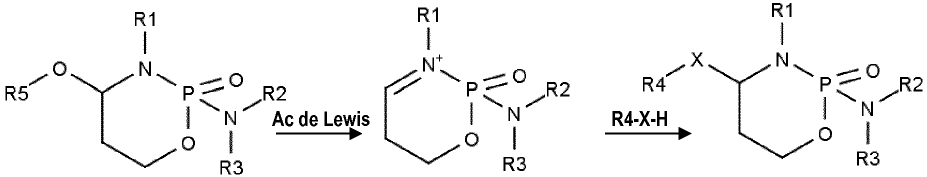

Ainsi, elle peut être décomposée en une étape de formation de l'iminium puis une étape de réaction avec le nucléophile R4-XH (également appelée amidoalkylation). L'acide de Lewis peut être notamment TMSOTf, AlCl₃ et BF_{3.}OEt₂. Selon l'invention, l'acide de Lewis est BF₃OEt₂. De préférence, l'acide de Lewis BF₃ est utilisé avec O(CH₂-CH₃)₂. Le ratio entre le nombre de moles de l'acide de Lewis et le nombre de moles du composé (II) est généralement inférieure 1,5. Un ratio molaire inférieur à 1,5 englobe un ratio molaire inférieur à 1,5 ; 1,4 ; 1,3 ; 1,2 ; 1,1 ; 1,0 ; 0,9 ; 0,8 ; 0,7 ; 0,6 et 0,5. En particulier, le ratio entre le nombre de moles de l'acide de Lewis et le nombre de moles du composé (II) peut être compris dans une gamme allant de 0,01 à 0,7, de préférence de 0,05 à 0,5. A titre d'exemple, la réaction peut être mise en oeuvre en présence de 0,05 Eq à 0,5 Eq de BF₃.OEt₂ quand R4-XH est le pentan-1-ol ou en présence d'environ 1,1 Eq quand R4-XH est le squalénol.

Bien que R5 puisse être un radical méthyle ou éthyle, le radical méthyle est préféré.

Le solvant doit être approprié pour dissoudre les composés de formule (II) et (III). Par exemple, en fonction des réactifs mis en jeu, le solvant peut être THF (tétrahydrofurane) ou CH₂Cl₂ (dichlorométhane). Il va de soi que l'on évitera l'utilisation d'un solvant nucléophile tel qu'un alcool qui peut rentrer en compétition avec le composé de formule (III) (c'est-à-dire R4-XH). Dans un mode de réalisation préférée, le solvant est du dichlorométhane (CH₂Cl₂₎.

La réaction peut être réalisée dans une gamme de température comprise entre -80°C et 0°C, de préférence débutant à environ -78°C. Le temps de réaction pourra être adapté selon les composés et peut par exemple être compris entre 30 minutes et 12 heures, de préférence environ 60 minutes.

De préférence, la fonction XH est une fonction alcool ou une fonction thiol primaire.

Les composés de formule (II) peuvent être préparés par oxydation anodique, notamment comme décrit dans l'article de Paci et al (2001, Bioorg Med Chem Lett, 11, 1347-1349). En particulier, ils peuvent être préparés par oxydation anodique dans du méthanol ou de l'éthanol avec une électrode de carbone graphite en présence de tétraéthylammonium tosylate (Et₄NOTs) ou de tétra fluoroborate de Tétraéthylammonium (TEABF4).

Comme cela est illustré dans les exemples, le procédé selon l'invention est particulièrement adapté pour la préparation de composés d'oxazaphosphorine de formule (II) dans laquelle R2 est H et R1 et R3 sont choisis indépendamment l'un de l'autre parmi -CH(CH₃)-CH₂-Cl et -(CH₂)₂-Cl.

Tout type de composés R4-XH peut être utilisé pour la mise en oeuvre du procédé selon l'invention. Les préférences concernant le groupe R4 sont présentées plus en détails ci-après.

On notera que les rendements du procédé selon l'invention sont généralement plus élevés que ceux obtenus par la méthode classique de synthèse consistant à oxyder électrochimiquement le composé d'oxazaphosphorine en présence de l'agent nucléophile R4-XH.

De manière surprenante, la Demanderesse a montré que la longueur de chaîne du groupe R4 n'est pas un critère limitant à la réaction puisqu'il est possible de greffer des dérivés alkyles de longueur de chaîne très différente tels que le pentanol ou le tris-nor squalénol avec des rendements et des durées de réaction similaires.

Dans un mode de réalisation particulier, les étapes de fourniture d'un composé de formule (II) et de réaction en présence d'un acide de Lewis avec un composé R4-XH peuvent se faire conjointement dans le même milieu réactionnel.

De manière alternative, les composés de formule (I) peuvent être préparés par une méthode directe d'oxydation anodique à condition que la taille du radical R4 soit limitée à 7 carbones. Cela ne constitue pas la méthode préférée puisque elle nécessite une grande quantité de nucléophile et impose l'utilisation de composés résistants aux conditions oxydantes. Dans ce mode de réalisation, le méthanol ou éthanol sera remplacé par le composé de formule R4-OH.

Il est également décrit des nouvelles molécules de formule (I) 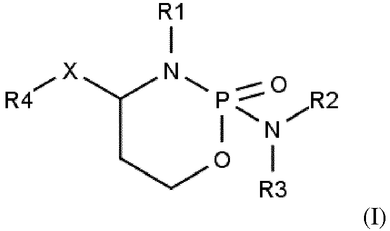 dans laquelle
X est O ou S ;
R1 et R2 sont, indépendamment, H, -CH(CH₃)-CH₂-Cl ou -(CH₂)₂-Cl, à condition qu'au moins un des deux soit -CH(CH₃)-CH₂-Cl ou -(CH₂)₂-Cl;
R3 est -CH(CH₃)-CH₂-Cl ou -(CH₂)₂-Cl ; et,
R4 est un radical de formulation ou de vectorisation d'un moins 3 carbones.

Lorsque R1 est H et R2 et R3 sont indépendamment -(CH₂)₂-Cl ou -CH(CH₃)-CH₂-Cl, le composé (I) ou (II) est un dérivé de cyclophosphamide. Dans certains aspects, R2 et R3 sont -(CH₂)₂-Cl.

Lorsque R1, R2 et R3 sont indépendamment -(CH₂)₂-Cl ou -CH(CH₃)-CH₂-Cl le composé (I) ou (II) est un dérivé de trofosfamide. Dans certains aspects, R1, R2 et R3 sont indépendamment -(CH₂)₂-Cl.

La présente invention concerne des dérivés de l'ifosfamide. Ainsi, dans les composés de formule (I) selon l'invention,, R2 est H et R1 et R3 sont indépendamment -(CH₂)₂Cl ou -CH(CH₃)CH₂Cl. Dans certains modes de réalisation, R1 et R3 sont-(CH₂)₂Cl.

Dans d'autres modes de réalisation, R1 et R3 sont -CH(CH₃)CH₂Cl.

Dans un mode de réalisation supplémentaire, R1 est -(CH₂)₂Cl et R3 est -CH(CH₃)CH₂Cl. Dans un autre mode de réalisation, R3 est -(CH₂)₂Cl et R1 est -CH(CH₃)CH₂Cl.

De manière générale, R4 peut comprendre un lien ou espaceur, ce lien ou espaceur étant situé à l'extrémité proximale et donc rattaché à la fonction X. Les liens et espaceurs sont bien connus de l'homme du métier, tels que des dérivés de la cystéine. Facultativement, ils permettent d'introduite la fonction XH. De préférence, la fonction XH est un alcool ou un thiol primaire.

Les radicaux de formulation ou de vectorisation sont bien connus dans le domaine. A titre d'illustration, nous pouvons citer les revues suivantes : Singh et al (2008, Curr Med Chem, 15, 1802-1826), Das et al (2009, Curr Opin Drug Deliv, 6, 285-304), etc...

Il est décrit que R4 pour les formules (I) et (III) est sélectionné parmi les radicaux suivants ou comprennent ces radicaux :
- un groupe hydrocarboné de 3 à 40 atomes de carbone, de préférence de 3 à 30 atomes de carbone (de préférence de 4, 5, 6, 7 ou 8 à 30 carbones), saturé ou insaturé, linéaire ou ramifié, éventuellement substitué par un ou plusieurs groupes sélectionnés parmi un groupe -OR, -C(O)OR, -OC(O)R, -OC(O)OR, -C(O)R, - NRR', -C(O)NRR', -NC(O)R, -NRC(O)R', -SR, un halogène, un cyano (-CN), un aryle, un hétéroaryle, arylalkyle ; R et R' étant un hydrogène ou un alkyle en C₁-C₃ ;
- un lipide ;
- un acide aminé ;
- un peptide ou une protéine, de préférence un peptide ;
- une vitamine ;
- un aptamère ;
- un polymère ; et,
- un radical polyol.

Par « alkyle en C₁-C₃ » est entendu un méthyle, éthyle, propyle ou isopropyle.

Par « halogène » est entendu un atome d'halogène sélectionné parmi Cl, Br, I et F.

Par « aryle » est entendu un radical hydrocarboné aromatique, substitué ou non, ayant de préférence 6 à 14 atomes de carbone. De préférence, les radicaux aryles selon la présente invention sont choisis parmi le phényle, le naphtyle (par exemple 1-naphtyle ou 2-naphtyle), le biphényle (par exemple, 2-, 3- ou 4- biphényle), l'anthryle ou le fluorényle. Les groupes phényles, substitués ou non, sont tout particulièrement préférés.

Par « hétéroaryle » est entendu un radical hydrocarboné aromatique comportant un ou plusieurs hétéroatomes tels que l'azote, le soufre et l'oxygène, substitué ou non, ayant de préférence 6 à 14 atomes de carbone. A titre d'exemple, on peut citer les groupements pyridinyle, pyridazinyle, pyrimidyle, pyrazyle, triazinyle, pyrrolyle, pyrazolyle, imidazolyle, (1,2,3)- et (1,2,4)-triazolyle, pyrazinyle, pyrimidinyle, tétrazolyle, furyle, thiényle, isoxazolyle, thiazolyle, isoxazolyle ou oxazolyle, etc...

Par « arylalkyle » est entendu un radical du type aryle substitué par un groupement alkyle. Les termes « alkyle » et « aryle » répondent aux définitions précédemment énoncées. Des exemples de groupes alkylaryle sont notamment tolyle, mésythyle et xylyle.

Lorsque R4 est un groupe hydrocarboné de 3 à 30 atomes de carbones, R4 est par exemple un alkyle ou alcène en C₃ à C₇, linéaire ou branché, comprenant facultativement un ou plusieurs groupes hydroxyles, ou un résidu hydroxy-acide en C₃ à C₇. De préférence, R4 est linéaire. Dans un mode de réalisation particulier, R4 peut être sélectionné parmi un pentyle, un hexyle, ou un heptyle, pouvant facultativement comprendre une ou plusieurs insaturations et être substitué par un ou plusieurs groupes hydroxyles. De préférence, R4 peut être sélectionné parmi un pentyle, un hexyle, ou un heptyle, pouvant facultativement comprendre une insaturation et être substitué par un groupe hydroxyle. De préférence, les groupes hydroxyles ne sont pas primaires, mais plutôt secondaire ou tertiaire.

Des composés particuliers sont les suivants :

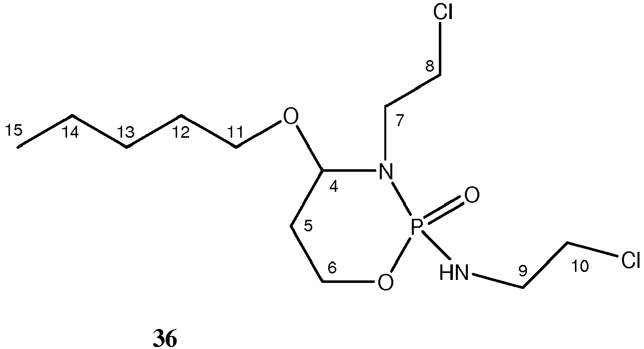

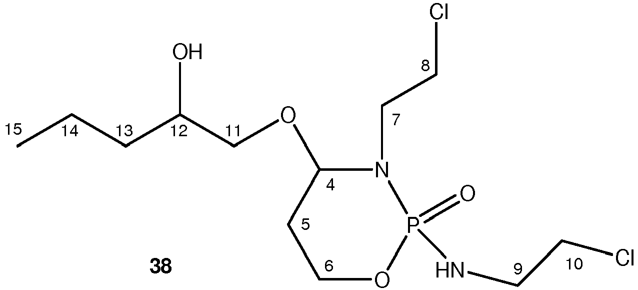

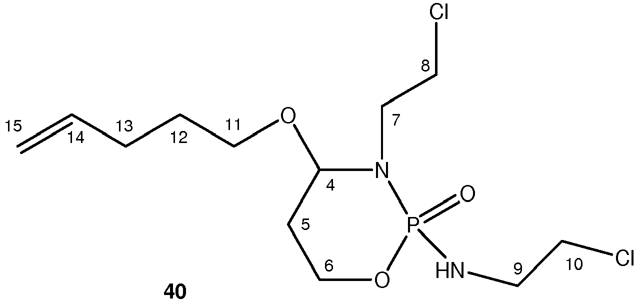

Lorsque R4 est un lipide, il peut être sélectionné parmi les acides gras, les éicosanoïdes, les acylglycérols, les phosphoacylglycérols, les sphingolipides, les stérols, et les saccharolipides. En particulier, R4 peut être un acide gras, saturé ou insaturé, linéaire ou branché. De préférence, l'acide gras présente une chaîne à au moins 18 carbones. Plus particulièrement, l'acide gras présente une chaîne insaturée et branchée à au moins 18 carbones Dans un mode de réalisation préféré de l'invention, R4 est un squalènoyle. En effet, le squalène présente un grand intérêt pour la formulation de médicament (WO2006/090029). De manière alternative, R4 peut être un stérol tel que cholestérol. On considère tout particulièrement les radicaux lipidiques permettant la formation de nanoparticules, de micelles ou de liposomes, en particulier dans un solvant polaire, de préférence une phase aqueuse. De tels lipides sont bien connus de l'homme du métier. On peut citer, de manière non exhaustive, le cholesterol, les phospholipides et les squalènoyles.

Lorsque R4 est un acide aminé, l'acide aminé peut être naturel ou non.

Lorsque R4 est un peptide, tout peptide connu pour permettre le ciblage de tissus ou de cellules, notamment des tumeurs et des cellules cancéreuses ou de la néovascularisation, souvent via des récepteurs cellulaires, est adapté à la présente invention. Ces peptides sont bien connus de l'homme du métier et comprennent les peptidomimétiques. A titre d'illustration, on peut citer ceux ciblant les peptides ou peptidomimétiques permettant de cibler les tumeurs (WO2008/120098, WO2000/032237) par exemple ceux comprenant le motif asparagine-glycine-arginine (NGR), glycine-serine-leucine (GSL) ou arginine-glycine-aspartate (RGD) (WO2008/045252, WO2006/095234, WO2005/123767, WO2002/026776, WO98/010795), ou ceux décrits dans la demande WO00/032237. En outre, des peptides ont été décrits permettant de cibler un organe particulier, par exemple le poumon (WO2003/105907), de traverser la barrière hémato-encéphalique (WO2003/070755, WO2003/059394, WO2003/026701, WO2003/026700, WO2002/067994, WO00/032236), de faciliter la pénétration cellulaire ou nucléaire du médicament ((WO2005/016960, WO2001/064738). R4 peut être également une lectine, EGF (facteur de croissance épidermique), ou un anticorps ou un fragment de celui-ci présentant le domaine de liaison à l'antigène, de préférence un antigène spécifique du cancer ou de l'organe ou du tissu ciblé.

Lorsque R4 est un radical polyol, on préfèrera un saccharide ou un polysaccharide. Par exemple, R4 peut être un glucose. De manière alternative, R4 peut être un chitosan.

Lorsque R4 est une vitamine, l'acide folique sera préféré. L'acide folique est couramment utilisé pour vectoriser un médicament, en particulier dans le domaine de l'oncologie. De manière alternative, R4 peut être également la vitamine B12.

Lorsque R4 est un aptamère, cet aptamère sera de préférence spécifique d'un antigène tumoral.

Lorsque R4 est un polymère, on préfèrera un polymère permettant la formation de nanoparticules. De tels polymères sont bien connus de l'homme du métier. On peut citer à titre d'illustration le poly(cyanoacrylate d'alkyle) tel que décrit dans WO1999/043359, un polyamine, un N-(2-hydroxypropyl)méthacrylamide (HPMA), un poly(éthylène glycol), un polymère d'acide lactique, ou un polyglutamate.

Selon la présente invention, R4 est représenté par la formule (IV) R₅(Y)ₐ dans laquelle Y représente un espaceur ; a est 0 ou 1 ; et R5 est un groupe hydrocarboné de 3 à 30 atomes de carbone, linaire ou ramifié, saturé ou insaturé, éventuellement substitué par un ou plusieurs OH.

Y est choisi parmi -(CH₂)ₘ-, -CONH(CH₂)ₘ-, NHCO(CH₂)ₘ-, -COO(CH₂)ₘ- et -OCO(CH₂)ₘ- avec m est un entier allant de 1 à 10
« a vaut 1 » signifie que le groupe R4 possède un espaceur et donc que Y est présent. « a vaut 0 » signifie que le groupe R4 ne possède pas d'espaceur et que Y est absent.

R5 peut comprendre 1, 2, 3 ou 4 insaturations pouvant être indépendamment une double ou une triple liaison. R5 peut également comprendre 1, 2, 3, 4, 5 ou 6 ramifications, lesdites ramifications étant de préférence un groupe méthyle.

Dans certains modes de réalisation, R5 est choisi parmi le groupe constitué par :
(i) un alkyle en C₂-C₉ éventuellement substitué par un hydroxyle ;
(ii) un alcène en C₂-C₉, de préférence, de formule -H₂C=CH(CH₂)ₙ- avec n un entier allant de 1 à 8 ; et
(iii) un groupe hydrocarboné comprenant un ou plusieurs motifs dérivés de l'isoprène, de préférence choisi parmi le groupe constitué par :
   (a) (CH₃)₂C=CH-CH₂-CH₂-[C(CH₃)=CH-CH₂-CH₂]ₘ- avec m un entier allant de 0 à 5 et
   (b) (CH₃)₂C=CH-CH₂-CH₂-[C(CH₃)=CH-CH₂-CH₂]ₚ-[CH=C(CH)₃-CH₂-CH₂]_{q}- avec p est un entier allant de 1 à 5 et q est un entier allant de 1 à 5

La ou les doubles liaisons présentes dans le radical R5 peuvent être indépendamment en conformation *trans* ou en conformation *cis.* Dans certains modes de réalisation, la ou les doubles liaisons sont en conformation *trans.*

Des exemples particuliers de composés selon l'invention pour lesquels R5 correspondent aux radicaux définis au point (i) ou (ii) ont été présentés ci-avant (voir les composés particuliers 36, 38 et 40). Dans certains modes de réalisation du composé selon l'invention, R4 est de formule R5(Y)ₐ dans laquelle :
▪ R5 est choisi parmi le groupe constitué par :
   (a) (CH₃)₂C=CH-CH₂-CH₂-[C(CH₃)=CH-CH₂-CH₂]ₘ- avec m un entier allant de 0 à 5 et
   (b) (CH₃)₂C=CH-CH₂-CH₂-[C(CH₃)=CH-CH₂-CH₂]ₚ-[CH=C(CH)₃-CH₂-CH₂]_{q}- avec p est un entier allant de 1 à 5 et q est un entier allant de 1 à 5 ;
▪ Y est choisi parmi -(CH₂)ₘ-, -CONH(CH₂)ₘ-, NHCO(CH₂)ₘ-, -COO(CH₂)ₘ- et -OCO(CH₂)ₘ- avec m est un entier allant de 1 à 10 ; et
▪ a est 0 ou 1.

Dans certains modes de réalisation particulier, R5 est un radical choisi parmi le groupe constitué par :
▪ (CH₃)₂C=CH-CH₂-CH₂-[C(CH₃)=CH-CH₂-CH₂]₂-[CH=C(CH)₃-CH₂-CH₂]₂-,
▪ (CH₃)₂C=CH-CH₂-CH₂-[C(CH₃)=CH-CH₂-CH₂]₂-[CH=C(CH)₃-CH₂-CH₂]-; et
▪ (CH₃)₂C=CH-CH₂-CH₂-[C(CH₃)=CH-CH₂-CH₂]₂-.

Dans un mode de réalisation préféré de l'invention, R4 est un radical squalènoyle, comprenant facultativement un espaceur. Au sens de l'invention, un radical squalènoyle est un radical comprenant le motif: (CH₃)₂C=CH-CH₂-CH₂-[C(CH₃)=CH-CH₂-CH₂]₂-[CH=C(CH)₃-CH₂-CH₂]₂-. Dans un mode de réalisation particulier, R4 est un radical squalènoyle de formule suivante :
(CH₃)₂C=CH-CH₂-CH₂-[C(CH₃)=CH-CH₂-CH₂]₂-[CH=C(CH)₃-CH₂-CH₂]₂-(Y)ₐ dans laquelle Y et a sont tels que définis ci-dessus.

Dans un mode particulier du procédé précédemment décrit, le composé R4-XH est le tris-nor-squalénol, également dénommé ici squalénol, ce qui signifie que R4-XH est un composé de formule R5-Y-XH dans laquelle :
R5 est (CH₃)₂C=CH-CH₂-CH₂-[C(CH₃)=CH-CH₂-CH₂]₂-[CH=C(CH)₃-CH₂-CH₂]₂-, Y est CH₂ et X est O.

Dans un autre mode particulier, le composé R4-XH est le radical squalènoyle lié à -C(O)NH-(CH₂)₂-SH (c'est-à-dire le composé (CH₃)₂C=CH-CH₂-CH₂-[C(CH₃)=CH-CH₂-CH₂]₂-[CH=C(CH)₃-CH₂-CH₂]₂-C(O)NH-(CH₂)₂-SH).

Les composés de formule (I) particulièrement préférés sont les suivants :
- R1 et R2 sont, indépendamment, H, -CH(CH₃)-CH₂-Cl ou -(CH₂)₂-Cl, à condition qu'au moins un des deux soit -CH(CH₃)-CH₂-Cl ou -(CH₂)₂-Cl;
- R3 est -CH(CH₃)-CH₂-Cl ou -(CH₂)₂-Cl ; et,
- X est O et R4 est un radical squalènoyle ; ou R4X est squalènoyl-C(O)NH-(CH₂)₂-S-.

Dans un mode de réalisation encore plus préféré, R2 est H et R1 et R3 sont choisis indépendamment parmi -CH(CH₃)-CH₂-Cl et -(CH₂)₂-Cl. Plus particulièrement, R2 est H et R1 et R3 sont -(CH₂)₂-Cl. Alternativement, R2 peut être H et R1 et R3 sont -CH(CH₃)-CH₂-Cl.

Des composés particuliers de l'invention sont les suivants : 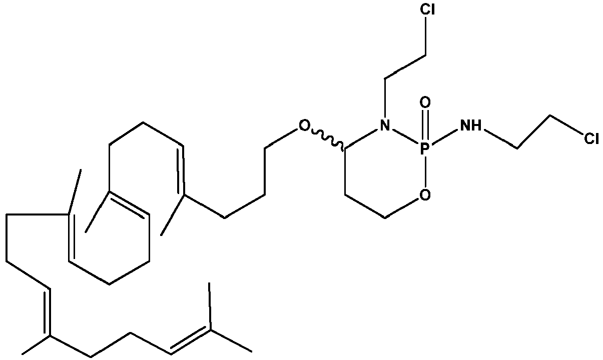 et/ou

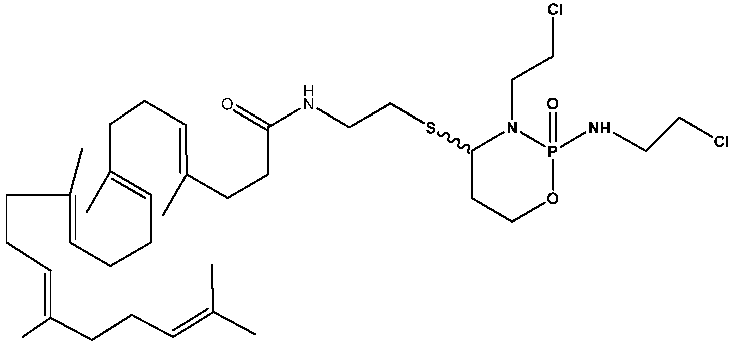

Par « environ » est entendu plus ou moins 10%, de préférence plus ou moins 5%.

Lorsque R4 est choisi de telle sorte qu'il est un radical, en particulier lipidique, permettant la formation de nanoparticules, de micelles ou de liposomes, la présente invention concerne également une nanoparticule formée par le composé de formule (I) de la présente invention. Dans un mode de réalisation préféré de l'invention, R4 est un squalènoyle, c'est-à-dire un radical comprenant le radical suivant : (CH₃)₂C=CH-CH₂-CH₂-[C(CH₃)=CH-CH₂-CH₂]₂-[CH=C(CH)₃-CH₂-CH₂]₂-,

Plus particulièrement, R2 est H et R1 et R3 sont -(CH₂)₂-Cl et R4 est un squalènoyle, X pouvant être O ou S. Alternativement, le composé de formule (I) peut présenter R2 étant H et R1 et R3 étant -CH(CH₃)-CH₂-Cl et R4 est un squalènoyle, X pouvant être O ou S. Dans un mode de réalisation tout particulièrement préférée, les composés seront choisis parmi les composés comprenant un radical squalénoyle tels que décrits ci-dessus. Les nanoparticules de composé de formule (I) peut être obtenues par solubilisation du composé dans un solvant organique comme l'acétone ou l'éthanol puis par ajout de ce mélange dans une phase aqueuse sous agitation conduit à la formation des nanoparticules en présence ou non de tensioactif(s). A titre de tensioactifs, on peut citer par exemple des copolymères de polyoxyéthylène-polyoxypropylène, des dérives phospholipidiques et des dérivés lipophiles de polyéthylène glycol. De préférence, les nanoparticules possèdent des tailles moyennes variant de 30-500 nm ou 70-200 nm.

Le composé peut être utilisé sous forme de tout sel pharmaceutiquement acceptable. Par sels pharmaceutiquement acceptables, on entend par exemple et de manière non limitative des sels d'addition basique ou acide pharmaceutiquement acceptables, hydrates, esters, solvates. L'expression sels pharmaceutiquement acceptables se réfère aux sels non toxiques, qui peuvent être généralement préparés en faisant réagir une base libre avec un acide organique ou inorganique convenable. Ces sels conservent l'efficacité biologique et les propriétés des bases libres. Comme exemples représentatifs de tels sels, on peut citer les sels hydrosolubles et hydroinsolubles, tels que les acétates, N-méthylglucamine ammonium, ansonates (4,4-diaminostilbènes-2,2'-disulfonates), benzènesulfonates, benzonates, bicarbonates, bisulfates, bitartrates, borates, bromhydrates, bromures, buryrates, camsylates, carbonates, chlorhydrates, chlorures, citrates, clavulariates, dichlorhydrates, diphosphates, édétates, édétates de calcium, édisylates, estolates, ésylates, fumarates, gluceptates, gluconates, glutamates, glycolylarsanylates, hexafluorophosphates, hexylrésorcinates, hydrabamines, hydroxynaphtoates, iodures, isothionates, lactates, lactobionates, laurates, malates, maléates, mandélates, mésylates, méthylbromures, méthylnitrates, méthylsulfates, mucates, napsylates, nitrates, 3-hydroxy-2-naphtoates, oléates, oxalates, palmitates, pamoates (1,1-méthylène-bis-2-hydroxy-3-naphtoates, ou emboates), pantothénates, phosphates, picrates, polygalacturonates, propionates, p-toluènesulfonates, salicylates, stéarates, subacétates, succinates, sulfates, sulfosalicylates, suramates, tannates, tartrates, téoclates, tosylates, triéthiodides, trifluoroacétates, valérates.

La présente invention concerne également une composition pharmaceutique comprenant un dérivé d'oxazaphosphorine de formule (I) ou une nanoparticule telle que défini précédemment. Cette composition pharmaceutique peut comprendre avantageusement un véhicule ou excipient pharmaceutiquement acceptable. Le véhicule pharmaceutiquement acceptable peut être choisi parmi les véhicules utilisés de manière classique selon chacun des modes d'administration. En fonction du mode d'administration prévu, les composés peuvent être sous forme solide, semi-solide ou liquide. Pour les compositions solides, telles que comprimés, pilules, poudres ou granulés à l'état libre ou inclus dans des gélules, la substance active peut être combinée avec : a) des diluants, par exemple le lactose, le dextrose, le sucrose, le mannitol, le sorbitol, la cellulose et/ou la glycine ; b) des lubrifiants, par exemple la silice, le talc, l'acide stéarique, son sel de magnésium ou de calcium et/ou le polyéthylène glycol ; c) des liants, par exemple le silicate de magnésium et d'aluminium, la pâte d'amidon, la gélatine, la gomme adragante, la méthylcellulose, la carboxyméthylcellulose sodique et/ou la polyvinylpyrrolidone ; d) des désintégrants, par exemple l'amidon, l'agar, l'acide alginique ou son sel de sodium, ou des mélanges effervescents ; et/ou e) des absorbants, colorants, aromatisants et édulcorants. Les excipients peuvent être par exemple du mannitol, lactose, amidon, stéarate de magnésium, saccharine sodique, talc, cellulose, glucose, sucrose, carbonate de magnésium et analogues de qualité pharmaceutique. Pour les compositions semi-solides, telles que suppositoires, l'excipient peut, par exemple, être une émulsion ou suspension grasse, ou à base de polyalkylène glycol, tel que le polypropylène glycol. Les compositions liquides, en particulier injectables ou à inclure dans une capsule molle, peuvent être préparées par exemple par dissolution, dispersion, etc. de la substance active dans un solvant pharmaceutiquement pur tel que, par exemple, l'eau, le sérum physiologique, le dextrose aqueux, le glycérol, l'éthanol, une huile et ses analogues.

Cette composition peut comprendre en outre un autre principe actif. Dans un mode particulier de réalisation, le principe actif supplémentaire est un agent anticancéreux. A titre d'exemples et de manière non-exhaustive, on peut citer notamment des interférons, le cisplatine, la bléomycine, le fluorouracile, le méthotrexate, la vincristine, l'actinomycine, la vinorelbine, les taxanes comme la paclitaxel et le docétaxel, ou une anthracycline. Par ailleurs, un principe actif destiné à contrecarrer la toxicité des oxazaphosphorines peut être ajouté à la composition pharmaceutique, notamment le mercaptoéthanesulfonate de sodium. Cette composition pharmaceutique peut également être utilisée en association avec la radiothérapie.

La composition de l'invention peut être administrée par toute voie adaptée et, de manière non limitative par la voie parentérale, comme par exemple, sous forme de préparations injectables par voie sous-cutanée, intraveineuse ou intramusculaire ; par voie orale (ou *per os*), comme par exemple, sous forme de comprimés enrobés ou non, de gélules, de poudres, de granulés, de suspensions ou de solutions orales (une telle forme pour l'administration par voie orale peut être soit à libération immédiate, soit à libération prolongée ou retardée) ; - voie rectale, comme par exemple, sous forme de suppositoires ; - voie topique, notamment transdermique, comme par exemple, sous la forme de patchs, de pommades ou de gels ; - voie intra-nasale, comme par exemple sous forme d'aérosols et de sprays ; - voie perlinguale ; - voie intraoculaire.

La composition pharmaceutique comprend typiquement une dose efficace d'un dérivé d'oxazaphosphorine de formule (I) de l'invention. Une «dose thérapeutiquement efficace » telle qu'elle est décrite ici s'entend comme la dose qui donne un effet thérapeutique pour une condition et un régime d'administration donnés. C'est typiquement la dose moyenne d'une substance active à administrer pour améliorer sensiblement certains des symptômes associés à une maladie ou à un état pathologique.

Une « dose thérapeutiquement efficace » d'une substance active n'est pas tenue de guérir une maladie ou un trouble mais fournira un traitement pour cette maladie ou ce trouble de façon à ce que son apparition soit retardée, entravée ou empêchée, ou que ses symptômes soient atténués, ou que son terme soit modifié ou, par exemple, soit moins sévère ou que la récupération du patient soit accélérée. Il est entendu que la « dose thérapeutiquement efficace » pour une personne en particulier dépendra de divers facteurs, incluant l'activité/efficacité de la substance active, son heure d'administration, sa voie d'administration, son taux d'excrétion et son métabolisme, les associations/interactions médicamenteuses et la sévérité de la maladie (ou du trouble) traitée à titre préventif ou curatif, ainsi que l'âge, le poids corporel, l'état de santé global, le sexe et/ou le régime alimentaire du patient.

Pour un traitement par voie systémique, on peut envisager d'administrer le composé de formule (I) à une dose d'environ 25 à 500 mg/kg de poids corporel et par jour, de préférence d'environ 25 à 300 mg/kg.

La présente invention porte également sur une composition pharmaceutique selon l'invention pour une utilisation dans le traitement du cancer ou comme immunosupresseur. Elle concerne également l'utilisation d'une composition pharmaceutique selon l'invention, d'un dérivé d'oxazaphosphorine de formule (I) ou d'une nanoparticule telle que défini précédemment pour la fabrication d'un médicament destiné au traitement du cancer ou d'un immunosuppresseur. Enfin, la présente invention concerne une méthode pour traiter un sujet ayant un cancer, la méthode comprenant l'administration d'une dose thérapeutiquement efficace d'une composition pharmaceutique selon l'invention, d'un dérivé d'oxazaphosphorine de formule (I) ou d'une nanoparticule telle que défini précédemment. La présente invention concerne aussi une méthode pour traiter un sujet nécessitant une immunosupression, la méthode comprenant l'administration d'une dose thérapeutiquement efficace d'une composition pharmaceutique selon l'invention, d'un dérivé d'oxazaphosphorine de formule (I) ou d'une nanoparticule telle que défini précédemment.

Le cancer peut être une tumeur solide ou un cancer hématopoïétique. Ainsi, le cancer peut être sélectionné parmi les leucémies chroniques, les leucémies aiguës lymphoïdes, la maladie de Hodgkin, les lymphomes hodgkiniens et non hodgkiniens, les cancers du poumon, du sein, de la prostate, de la vessie et de l'ovaire, les sarcomes, les neuroblastomes, les myélomes ; les mélanomes,....

D'autres aspects et avantages de la présente invention apparaîtront à la lecture des exemples ci-dessous, qui sont de nature illustrative seulement mais ne limitent pas la portée de la présente demande.

### Exemple 1 : Préparation d'analogues 4-substitués de l'IFO (Ifosfamide)

L'oxydation anodique dans le méthanol de l'ifosfamide permet d'accéder à une entité chimique (le 4-MeO-IFO) dont le pouvoir cytotoxique est équivalent à celui du métabolite actif de l'IFO. Cependant, ce 4-MeO-IFO est assez instable d'un point de vue chimique, libérant la moutarde isophosphoramide rapidement. Afin de moduler cette cinétique de libération et de faciliter la pénétration intracellulaire de cette petite molécule très polaire, les inventeurs ont envisagé l'insertion sur cette position 4 de chaînes aliphatiques plus longues. Ce concept constitue le premier pas vers la modification de l'ifosfamide pour l'insérer dans une forme vectorisée lipophile, tel un liposome ou un système nanoparticulaire.

### Substitution par voie directe

L'oxydation de l'ifosfamide génère un iminium. Celui-ci est piégé par le nucléophile présent dans le milieu, aboutissant à une oxazaphosphorinane 4-substituée. L'objet de ce descriptif est l'étude de la faisabilité de cette voie synthétique et l'évaluation des substrats et de la quantité minimale compatible avec cette voie directe.

### Oxydation électrochimique de l'ifosfamide en présence de pentan-1-ol :

Dans un premier temps, les inventeurs ont utilisé l'alcool amylique primaire pour étudier la faisabilité de cette réaction en présence d'un alcool à longue chaîne aliphatique.

Afin de définir un mode opératoire le plus efficace possible, les inventeurs ont fait varier les conditions expérimentales de la réaction décrite sur le Schéma 1.

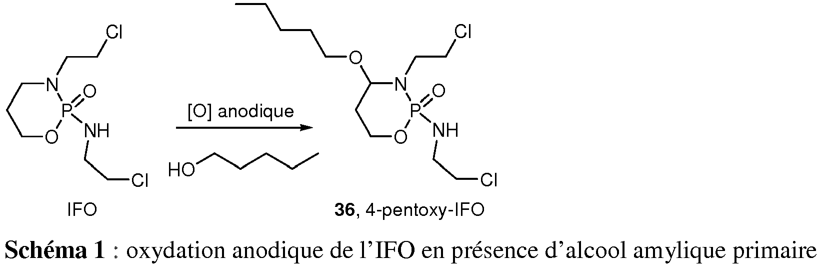

Une électrolyse de l'IFO a été réalisée, en présence de pentan-1-ol, dans l'acétonitrile, à intensité constante, grâce à des électrodes en graphite. Le tétraéthylammonium tétrafluoroborate (TEABF₄) était l'électrolyte support utilisé. La réaction a été suivie par chromatographie sur couche mince (CCM) et arrêtée lorsque tout l'IFO est consommé. A l'arrêt de la réaction, du bicarbonate de sodium a été ajouté afin de neutraliser l'acidité électrogénérée. Les produits obtenus, isolables, ont ensuite été purifiés par chromatographie sur colonne.

L'IFO est un mélange racémique du fait de la chiralité portée par le phosphore. Comme pour la méthoxylation, la pentoxylation a génèré 4 diastéréomères, énantiomères deux à deux.

Par nature, les énantiomères ne pouvaient pas être physiquement différenciés. Par contre, les déplacements chimiques en ³¹P-RMN des deux couples de diastéréomères étaient distincts.

Les proportions des divers produits ont donc été déterminées par ³¹P-RMN.

Par analogie avec la méthoxylation, il était possible d'attribuer les signaux en ¹H-RMN à l'un ou l'autre des formes diastéréomériques : en effet, la fixation d'un groupe alkoxy sur le carbone en α de l'azote se traduit par un groupe de 3 signaux correspondant aux hydrogènes portés par les C4 et C6, et qui diffèrent d'un diastéréomère à l'autre.

### Influence de la quantité d'alcool

L'électrolyse a été réalisée en variant la quantité d'alcool introduite. Pour une quantité donnée d'électrolyte support (1 Eq.), les résultats sont regroupés dans le Tableau 1.

**Tableau 1 : influence de la quantité de nucléophile**

| **Essai** | **Eq. de Pentanol** | **Intensité (mA)** | **Arrêt de la réaction** | **Rendement (en %)** |
|---|---|---|---|---|
| A | 9 | 20 | 3,75 F/mol | < 15 |
| B | 15 | 20 | 3,60 F/mol | 34 |
| C | 30 | 20 | 3,75 F/mol | 41 |

Dans un premier temps, au moins 15 équivalents d'alcool semblent nécessaires pour obtenir un rendement satisfaisant (calculé par rapport à l'IFO). Par contre, le gain observé lorsque l'on passe de 15 à 30 équivalents était peu élevé.

### Oxydation anodique de l'IFO en présence de différents alcools

Il s'agissait ici d'étudier, en utilisant les conditions mises au point avec le pentan-1-ol, la compatibilité de fonctions portées par l'alcool avec le processus électrochimique.

La réaction générale est présentée sur le Schéma 2.

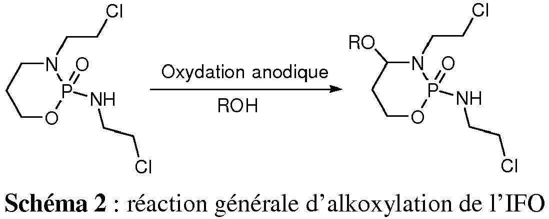

L'IFO était donc oxydé par voie électrochimique, en présence de différents alcools à 5 atomes de carbone, dans l'acétonitrile. La réaction a été arrêtée lorsque tout le produit de départ semblait consommé par suivi CCM. A la fin de la réaction, du bicarbonate de sodium (1 Eq.) a été ajouté au milieu pour neutraliser les hydrons électrogénérés. Les proportions des divers diastéréomères ont été déterminées sur le brut réactionnel par ³¹P-RMN avant d'être isolés par « flash-chromatographie ».

Les résultats de ces différentes manipulations sont décrits dans le Tableau 2.

**Tableau 2 : résumé des différents essais d'alkoxylation**

| **Alcool** | **Arrêt (F/mol)** | **Résultats Proportions des diastéréomères** | **Rendement global (%)** |
|---|---|---|---|
| Pentan-1-ol | 3,6 | 80/20 | 34 |
| Pentan-3-ol | 2,7 | 55/45 | 0* |
| Pentan-1,2-diol | 2,5 | 52/48 | 22 |
| Pentan-1,4-diol | 3,5 | 53/47 | 0* |
| 4-Pentèn-1-ol | 2,9 | ND | 19 |
| Heptan-1,7-diol | 2,7 | 80/20 | 27 |

| | | | |
|---|---|---|---|
| * produits non isolés. | | | |

Dans un premier temps, on a pu remarquer qu'un alcool secondaire semble plus difficile à fixer qu'un alcool primaire sur l'IFO. Effectivement, les spectres RMN du brut d'électrolyse ne montraient que des traces d'alkoxylation. Les produits alkoxylés formés en faibles quantités n'étaient pas isolables. L'encombrement stérique gênerait l'accès de l'alcool à l'iminium de l'IFO.

De plus, dans le cas d'une compétition entre un alcool primaire et secondaire, la fixation était presque exclusivement réalisée par l'alcool primaire, le reste de la chaîne étant inchangé. Au cours des manipulations avec les diols, les spectres RMN des bruts réactionnels ont montré la présence de produits compatibles avec les produits d'addition attendus. Ceci a pu être confirmé dans le cas du pentan-1,2-diol. En revanche, malgré une mise en évidence sur le brut réactionnel, les produits n'ont pu être purifiés dans le cas du pentan-1,4-diol.

L'utilisation d'un composé comportant deux fonctions alcool primaire permet, tout en conservant un rendement équivalent de diminuer le nombre d'équivalents à 10 Eq. (comparés aux 15 utilisés pour les autres alcools). Dans les conditions utilisées, les formes ayant fixé deux alcools n'ont pas été caractérisées.

Ces résultats permettaient d'envisager la fixation de polyols sélectivement par une fonction alcool primaire. C'était un premier pas vers la fixation d'un ose sur l'IFO, dans le cadre d'une vectorisation.

La présence d'une double liaison C=C n'a pas empêché la fixation de l'alcool primaire à l'IFO et n'était pas modifiée lors de la réaction électrochimique.

### Compatibilités de différentes fonctions avec le protocole d'oxydation directe

Outre l'utilisation d'une grande quantité de nucléophile, le protocole d'oxydation directe impose l'utilisation de composés résistants aux conditions oxydantes.

### Amines

Les amines sont oxydables dans les conditions utilisées. Ainsi, dans ces conditions, et en présence d'ifosfamide, il y a oxydation concomitante des deux composés (IFO et amine).

### Thiol

Dans des conditions oxydantes, les thiols se dimèrisent en formant un pont disulfure (Schéma 3).

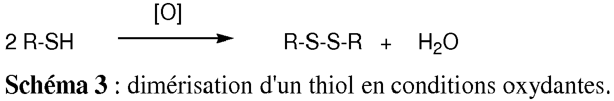

Le centre nucléophile (l'atome de soufre) n'est plus disponible pour la substitution de l'iminium.

### Conclusions et perspectives

Le protocole d'oxydation anodique de l'IFO en présence de 15 équivalents d'alcool est possible. Il permet d'accéder simplement à de nouveaux composés, jamais décrits. Ce protocole est également applicable à d'autres oxazaphosphorines comme le cyclophosphamide et le trofosfamide. Pour optimiser cette réaction, il est nécessaire d'utiliser un milieu réactionnel anhydre, afin d'éliminer un nucléophile concurrent.

Cependant, les inconvénients de cette voie directe sont multiples.

Le recours à 15 Eq de nucléophile pose plusieurs problèmes : cette méthode est limitée à des réactifs peu coûteux, et cet excès de réactif gène la purification de produits sensibles. Enfin, l'utilisation de composés nucléophiles possédant des fonctions oxydables est impossible.

### Substitution par voie indirecte

### Iminium piégé puis régénéré

Ce mode comprend 3 temps :
- la méthoxylation anodique,
- l'amidoalkylation, décomposée en
   o la régénération de l'iminium puis
   o la réaction *in situ* avec le nucléophile.

Cette séquence réactionnelle à 3 étapes est réalisée dans 2 milieux réactionnels (Schéma 4).

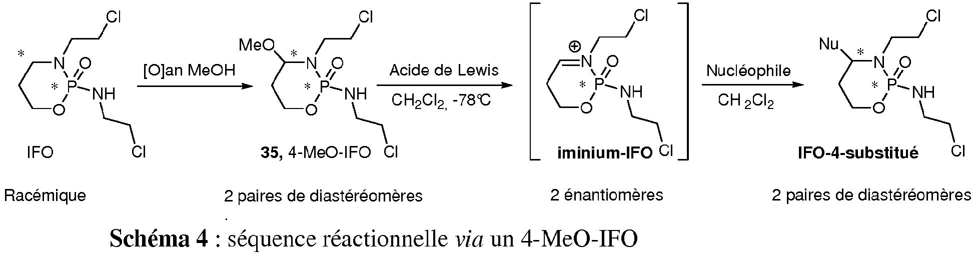

En pratique, l'ifosfamide subit une électrolyse dans le méthanol, puis le dérivé 4-MeO-IFO est mis en présence d'un acide de Lewis dans un solvant non nucléophile afin de régénérer l'iminium sur lequel réagit le nucléophile.

Cette technique permet d'utiliser des nucléophiles incompatibles avec l'oxydation anodique. Par comparaison avec la voie directe, l'autre avantage de cette technique est l'utilisation d'un nombre d'équivalents de nucléophiles plus faible.

L'iminium a été électrogénéré par oxydation anodique dans le méthanol et donc piégé sous forme de 4-MeO-IFO.

Une fois l'ifosfamide totalement consommé (passage de plus de 2,5-3,0 F/mol), le solvant a été évaporé en présence d'une base, le carbonate de sodium (Na₂CO₃), chargée d'éviter une acidification du milieu. On a repris le résidu par du diéthyl éther puis une simple filtration a donné accès aux produits méthoxylés. Lorsqu'il reste un peu de produit de départ, ou bien si la séparation des diastéréomères est souhaitée, une colonne chromatographique est nécessaire.

Finalement, de nombreuses méthoxylations sur des quantités variées d'IFO, allant de quelques dizaines de milligrammes à quelques grammes ont été réalisées. Dans une cellule électrochimique de 15 mL, l'oxydation de 2 grammes d'IFO a par exemple été réalisée. Les dérivés méthoxylés ont été obtenus avec un rendement de 82% (soit 1,8 g), après passage de 4,0 F.mol⁻¹. Ainsi cette réaction électrochimique semble adaptée à une utilisation synthétique : elle est relativement simple à mettre en oeuvre même si sa purification nécessite un certain savoir-faire. De plus, les produits obtenus sont relativement stables et peuvent être stockés plusieurs semaines au congélateur sous atmosphère inerte.

Sous l'action d'un acide de Lewis, le groupement méthoxyle peut régénérer l'iminium qui va être piégé par une grande variété de nucléophiles pour donner accès à des structures diverses. Est appelée amidoalkylation la réaction consistant à régénérer l'ion iminium à partir du dérivé méthoxylé puis à réaliser une addition nucléophile sur cet iminium.

La régénération de l'intermédiaire iminium à partir du dérivé méthoxylé se fait par l'intermédiaire d'un acide de Lewis. On a utilisé plutôt les acides de Lewis : BF₃.OEt₂, TiCl₄, Yb(OTf)₂, TMSOTf, pour les plus classiques.

L'acide de Lewis est impliqué simplement dans la régénération de l'iminium à partir du dérivé méthoxylé et non pas dans la diastéréosélectivité de l'addition.

### Electrolyse - méthoxylation par oxydation anodique

Angelo Paci et Thierry Martens ont décrit l'oxydation anodique de l'IFO et du CPM dans une cellule monocompartimentale (Paci et al. 2001b) (Schéma 5).

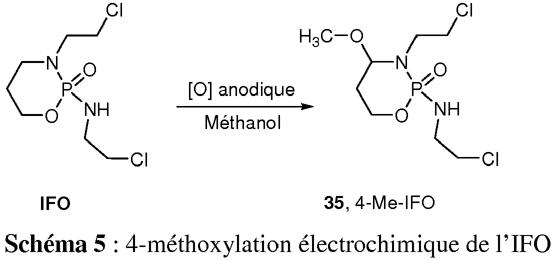

Afin d'obtenir un produit d'arrivée connu, l'étude a porté sur la séquence suivante, faisant intervenir le pentan-1-ol comme nucléophile (Schéma 6).

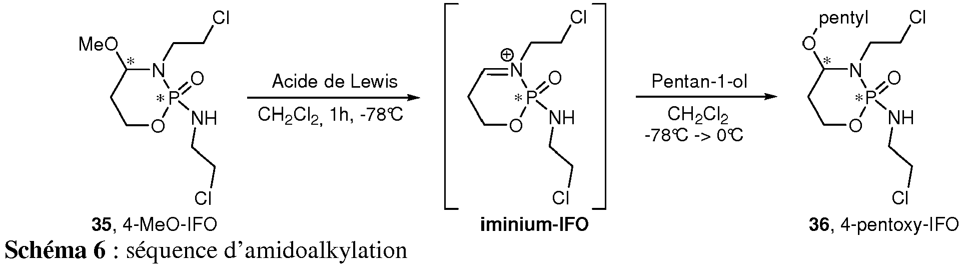

Le 4-MeO-IFO a été mis en présence d'un acide de Lewis à basse température (-78°C), pendant une heure (de manière arbitraire). Ensuite, le nucléophile (1 Eq. d'alcool amylique) est introduit dans le milieu qui est placé à 0°C pendant 15 minutes. Comme les pentoxy-IFO ont déjà été identifiés, un simple spectre ³¹P RMN a permis de déterminer rapidement l'efficacité de la réaction, tel que résumé dans le Tableau 3.

**Tableau 3 : influence de l'acide de Lewis utilisé sur l'amidoalkylation**

| **Acide de Lewis** | | **Résultats** | |
|---|---|---|---|
| *Nature* | *Quantité (en Eq)* | *Conversion (en %)* | *Proportion dias (9,6* / *9,3 ppm)* |
| TMSOTf | 0,1 | 30 (+ dégradation) | 85/15 |
| | 0,5 | Dégradation | ND |
| | 1 | Dégradation | ND |
| Ti(OEt)₄ | 0,1 | 0% | Reste 4-MeO-IFO & produits de dégradation |
| | 0,5 | 0% | |
| | 1 | 0% | |
| BF₃.OEt₂ | 0,1 | 59 | 82/18 |
| | 0,5 | 48 | 80/20 |
| | 1 | Dégradation | ND |

Il apparaît que le Ti(OEt)₄ ne semble pas être suffisamment réactif pour régénérer l'iminium à partir du 4-MeO-IFO. D'autre part, le TMSOTf semble quant à lui induire la dégradation du 4-MeO-IFO, sauf lorsqu'il est utilisé en quantité catalytique (10%mol) qui permet de former les diastéréomères avec un rendement médiocre. L'acide de Lewis utilisé ne semble pas modifier la diastéréosélectivité de la réaction, il agit plutôt sur le rendement et la stabilité de l'iminium intermédiaire généré, contribuant à la propreté du brut réactionnel. Au cours de la présente étude, il est apparu que l'éthérate de trifluorure de bore utilisé en quantités catalytiques (BF₃.OEt₂) est le plus efficace des acides de Lewis testés.

Les conditions (0,1 Eq. de BF₃.OEt₂, 1 heure à -78°C dans le CH₂Cl₂) déterminées ci-dessus ont été utilisées par la suite pour évaluer l'influence de la quantité de pentan-1-ol sur la conversion. Les résultats obtenus sont résumés dans le Tableau 4.

**Tableau 4 : influence du temps de réaction de l'iminium**

| **Pentan-1-ol** | | **Résultats** | |
|---|---|---|---|
| Quantité (en Eq.) | Temps (en min) | Conversion (en %) | Proportion dias (9,6 / 9,3 ppm) |
| 1 | 15 | 59 | 82/18 |
| 2 | 15 | 60 | 81/19 |
| 2 | 30 | 64 | 85,4/14,6 |
| 2 | 45 | 62 | 83,5/16,5 |
| 2 | 120 | 61 | 82/18 |

La quantité de pentan-1-ol ne semble influencer ni le taux de conversion ni la proportion des diastéréomères formés. Malgré des taux de conversion comparables, il semblerait que le temps de réaction optimum serait d'une demi-heure, avec une conversion de 64% et une proportion de diastéréomères de 85,4/14,6.

Sur quelques expériences, Les inventeurs ont vérifié que l'utilisation de THF à la place du dichlorométhane ne modifiait pas les résultats observés.

Les nucléophiles concurrents sont le méthanol généré en même temps que l'iminium et l'eau résiduelle présente dans le milieu. Les inventeurs ont tenté d'ajouter du tamis moléculaire activé 4Å afin de séquestrer l'eau résiduelle et le méthanol libéré. Cet ajout ne semble pas améliorer notablement les rendements observés.

Le greffage d'autres substrats tels que des amines, des composés poly fonctionnalisés ou des lipides est possible, et a été envisagée pour permettre de proposer la formation d'analogues pré-activés de l'IFO ciblés et vectorisés vers les tumeurs par greffage d'acides gras ou autres lipides, d'un peptide ou d'un sucre.

A titre d'exemple, l'application la plus récente et la plus prometteuse concerne la fixation d'un résidu squalènique par cette méthode en additionnant le squalènol ou le squalène-thiol en présence de BF₃, ET₂O sur le phosphoryl-iminium produit à partir du 4-méthoxy-IFM (Schéma7 et 8). Les composés formés, le SQ-4-*O*-IFM et le SQ-4-*S*-IFM, présentent deux propriétés intéressantes. La première est qu'ils constituent des formes pré-activées de l'IFM du fait de l'oxydation en 4 capable de libérer, en milieu aqueux légèrement acide, la moutarde alkylante directement sans activation métabolique. La seconde, est qu'ils sont capables de s'auto-assembler en milieu aqueux sous la forme de nanoparticules d'une taille d'environ 160 nm.

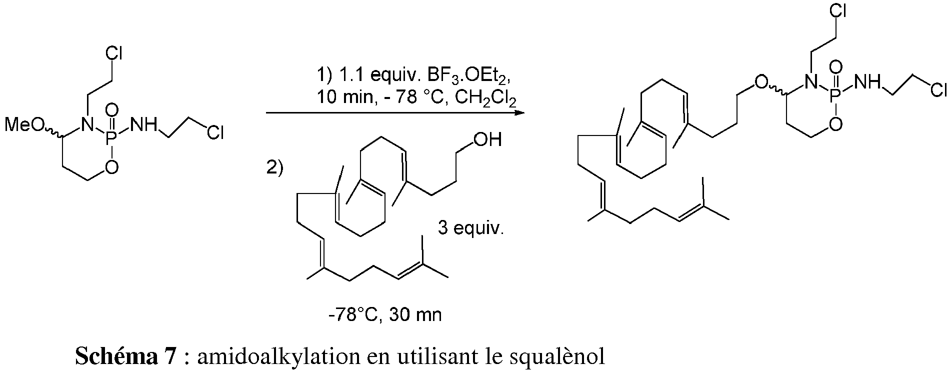

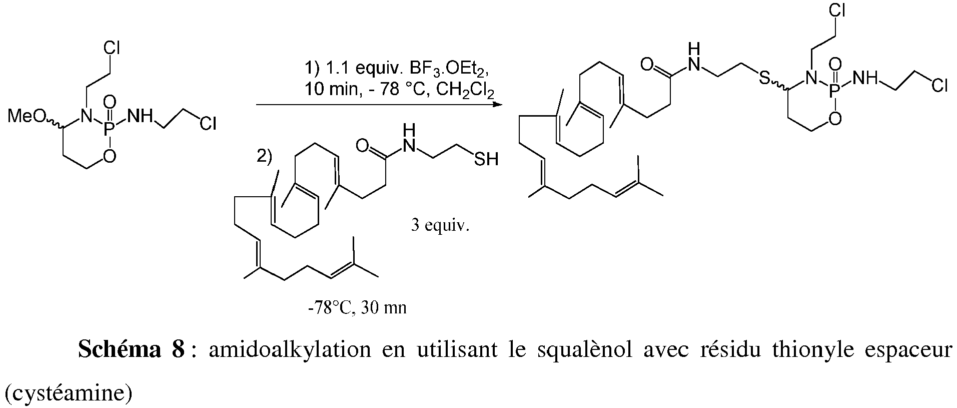

Le même type de réaction a été envisagé pour le cyclophosphamide ou le trofosfamide qui est également oxydable en position 4 sous sa forme 4-méthoxy pour conduire à la forme imine du phosphoryl.

Cette méthode en deux temps, permet d'obtenir des analogues de l'ifosfamide 4-substitués. Par comparaison à l'oxydation directe, l'avantage principal de cette technique est l'utilisation de quantités faibles de nucléophiles. Cet avantage sera majeur lors de l'utilisation de nucléophiles coûteux. De plus, cette technique est compatible avec une plus grande variété de nucléophiles. Enfin, la production de 4-MeO-IFO est réalisable sur de grandes quantités d'IFO.

### Analyste structurale

### (2-Chloro-ethyl)-[3-(2-chloro-ethyl)-2-oxo-4-pentyloxy-2λ5-[1,3,2]oxazaphosphinan-2-yl]-amine ou 4-pentoxy-IFO

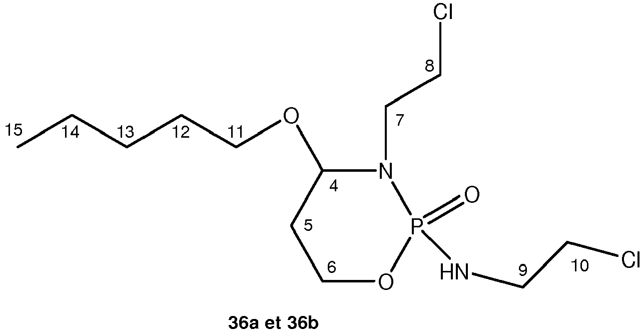

Protocole : Dans une cellule électrochimique de 8 mL, 110 mg d'IFO (0,4 mmol) ont été dissous dans 4,0 mL d'acétonitrile anhydre. Un équivalent d'électrolyte support (95 mg de tétra fluoroborate de tétraéthylammonium) puis quinze équivalents (7,1 mmol, 0,77 mL) de pentan-1-ol ont été ajoutés. Le milieu a été dégazé par bullage d'azote et placé dans un bain de glace. L'agitation a été assurée par un agitateur magnétique. Deux électrodes ont été introduites dans la cellule.

Après le passage de 3,6 F/mol sous 20 mA, la réaction a été stoppée, par coupure du courant électrique. Un équivalent de bicarbonate de soude a été ajouté au milieu afin de neutraliser les hydrons libérés au cours de la réaction et qui sont susceptibles de dégrader le produit.

Le solvant a été évaporé sous pression réduite, et le résidu a été repris 2 fois par 8 mL d'acétonitrile afin d'entraîner le maximum d'alcool. 15 mL d'éther éthylique ont été ensuite ajoutés afin d'insolubiliser l'électrolyte support. Après filtration de la phase éthérée, celle-ci a été évaporée sous pression réduite.

Les produits obtenus ont été purifiés par chromatographie (éluant éther éthylique / méthanol ; 95/5). Les diastéréomères **36a** et **36b** ont été obtenus avec une séparation de 90%.
Organoleptique : huile légèrement jaune
   *Rf* = 0,24 et 0,39 pour X1 et X2 (Et₂O).
*Formule brute :* C₁₂H₂₅Cl₂N₂O₃P.

*RMN* dans CDCl₃
*³¹**P*** δ (ppm) : **9,6** (diastéréomère **36a**), **9,3** (diastéréomère **36b**).
*¹³**C*** δ (ppm) : **13,9** (C₁₅) ; **22,4** (C₁₄) ; **28,4** (C₁₃) ; **29,5** (C₅) ; **42,7** (C₇) ; **44,0** (C₈) ; **46,6** (C₁₀) ; **49,5** (C₉) ; **62,5** (C₁₁) ; **68,5** (C₆) ; **89,3** (C₄).
*¹****H*** δ (ppm) : **0,92** (t, 3H, H₁₅) ; **1,40** (m, 4H, H₁₃, H₁₄) ; **1,62** (m, 2H, H₁₂) ; **2,10** (m, 1H, H_{5 Eq.}) ; **2,26** (td, 1H, H_{5 Ax.}) ; **3,33** (m, 2H, H₉) ; **3,45** (m, 2H, H₇) ; **3,56** (m, 2H, H₁₁) ; **3,73** (m, 4H , H₁₀, H₈) _{;} **4,15** (m, 1H, H_{6 Eq.}) ; **4,45** (m, 1H, H_{6 Ax.}) ; **4,60** (m, 1H, H₄).

### 1-[3-(2-Chloro-ethyl)-2-(2-chloro-ethylamino)-2-oxo-2,λ⁵-[1,3,2]oxazaphosphinan-4-yloxy]-pentan-2-ol / 4-(2OH)-pentoxy-IFO

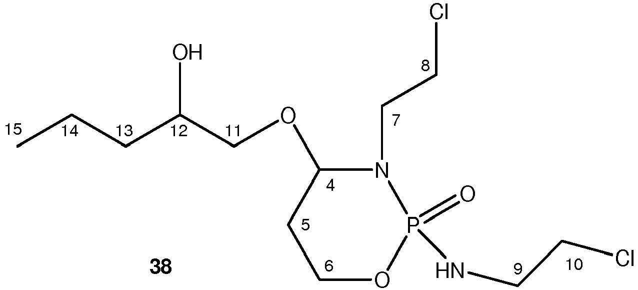

Protocole similaire à celui utilisé pour le 4-pentoxy-IFO

Les produits obtenus ont été purifiés par chromatographie (éluant éther éthylique / méthanol ; 90/10), 2 colonnes successives.
Organoleptique : huile légèrement jaune.
   *Rf* = 0,32 et 0,50 pour X1 et X2 respectivement (tâches peu distinctes) (Et₂O / MeOH 90/10).
*Formule brute :* C₁₂H₂₅Cl₂N₂O₄.

*RMN* dans CDCl₃.
*³¹**P*** δ (ppm) : **9,78** (diastéréomère **38a**), **9,88** (diastéréomère **38b**).
*¹**H*** δ (ppm) : **0,95** (t, 3H, H₁₅) ; **1,30** (sl, 1H, OH) ; **1,40** (m, 4H, 2H₁₃, 2H₁₄) ; **1,92** (m, 2H, H₅) ; **2,25** (sl, 1H, NH) ; **3,30** (m, 4H, 2H₇, 2H₉); **3,40** (m, 3H, 2H₁₁, H₁₂) ; **3,70** (m, 4H, 2H₈, 2H₁₀) ; **4,15** (m, 1H, H_{6Eq.}) ; **4,50** (m, 1H, H_{6Ax.}) ; **4,70** (m, 1H, H₄).

### (2-Chloro-ethyl)-[3-(2-chloro-ethyl)-2-oxo-4-pent-4-enyloxy-2λ⁵-[1,3,2] oxazaphosphinan-2-yl]-amine / ou 4-pentènoxy-IFO

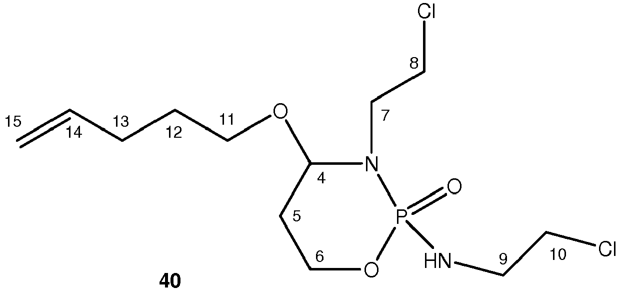

Protocole similaire à celui utilisé pour le 4-pentoxy-IFO.

Les produits obtenus sont purifiés par chromatographie (éluant éther éthylique / méthanol ; 97/3).
Organoleptique : huile légèrement jaune.
   *Rf* = 0,68 et 0,78 pour X1 et X2 respectivement (tâches peu distinctes) (Et₂O / MeOH 95/5).
*Formule brute :* C₁₂H₂₃Cl₂N₂O₃.
*IR (film)* ; ν (cm⁻¹) : 2924 (C-H), 2361, 1640 (C=C), 1434 (P-N), 1257 (P=O), 1065-1113 (P-O-C).

*RMN* dans CDCl₃.
*³¹**P*** δ (ppm) : **9,51** (diastéréomère **40a**), **9,23** (diastéréomère **40b**).
*¹³**C*** δ (ppm) : **28,9** (C₅) ; **35,6** (C₁₂) ; **36,9** (C₁₃) ; **42,6** (C₉) ; **44,5** (C₇) ; **47,3** (C₈) ; **50,0** (C₁₀) ; **62,5** (C₆) ; **67,8** (C₁₁) ; **89,5** (C₄) ; **115,9** (C₁₅) ; **138,0** (C₁₄).
*¹**H*** δ (ppm) : **0,95** (sl, 1H, NH) ; **1,30** (m, 2H, 2H₁₃) ; **1,48** (s, 1H, 1H₁₁) ; **1,75** (q, 2H, H₁₂) ; **1,90** (d, 1H, H_{11'}) ; **2,25** (m, 2H, 2H₅) ; **3,20** (m, 2H, 2H₉) ; **3,35** (m, 2H, 2H₇) ; **3,65** (m, 2H, 2H₈) ; **3,75** (m, 2H, 2H₁₀) ; **4,25** (m, 1H, H_{6Eq.}) ; **4,50** (m, 1H, H_{6Ax.}) ; **4,65** (m, 1H, H₄) ; **5,10** (m, 2H , 2H₁₅); **5,80** (m, 1H, H₁₄).

### (2-Chloro-ethyl)-[3-(2-chloro-ethyl)-2-oxo-4-squalenyl-[1,3,2]oxazaphosphinan-2-yl]-amine / ou 4-SQ-IFO

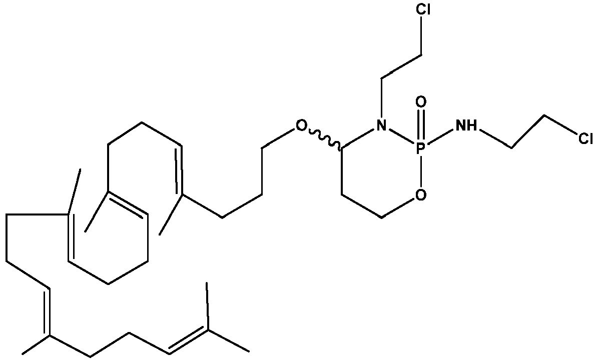

Protocole : A partir du 4-méthoxy-ifosfamide, est ajouté dans des conditions similaires aux produits précédents (-78°C, CH₂Cl₂, BF₃, EtO₂, 30 min) 1 équivalent de tris-nor-squalènol pour former le composé attendu avec un rendement de 53%.

Les produits obtenus sont purifiés par chromatographie.
Organoleptique : huile légèrement jaune.
*Formule brute :* C₃₄H₆₁Cl₂N₂O₃P
*IR (film)* ; ν (cm⁻¹) : 2956 (C-H), 2352, 1638 (C=C), 1432 (P-N), 1258 (P=O), 1063-1109 (P-O-C).

*RMN* dans CDCl₃.
*¹³**C*** δ (ppm) : **33,4** (C₅) ; **35,6** (C₁₂) ; **36,9** (C₁₃) ; **42,6** (C₉) ; **44,5** (C₇) **47,3** (C₈) ; **50,0** (C₁₀) ; **63,5** (C₆) ; **67,8** (C₁₁); **89,5** (C₄) ; **125,9** (C_{ethylen.}) ; **132,0** (Cquat).
*¹**H*** δ (ppm) : **0,95** (sl, 1H, NH) ; **1,30** (m, 2H, 2H₁₃) ; **1,55** (s, 18H) ; **1,95** (m, 20H) ; **3,20** (m, 2H, 2H₉) ; **3,35** (m, 2H, 2H₇) ; **3,55** (m, 2H, 2H₈) ; **3,65** (m, 2H, 2H₁₀) ; **4,25** (m, 1H, H_{6Eq.}) ; **4,50** (m, 1H, H_{6Ax.}) ; **4,65** (m, 1H, H₄) ; **5,05** (m, 5H , H_{ethyleniques}).

### (2-Chloro-ethyl)-[3-(2-chloro-ethyl)-2-oxo-4-mercaptoethylamidosclualen-[1,3,2] oxazaphosphinan-2-yl]-amine ou 4-thio SQ-IFO

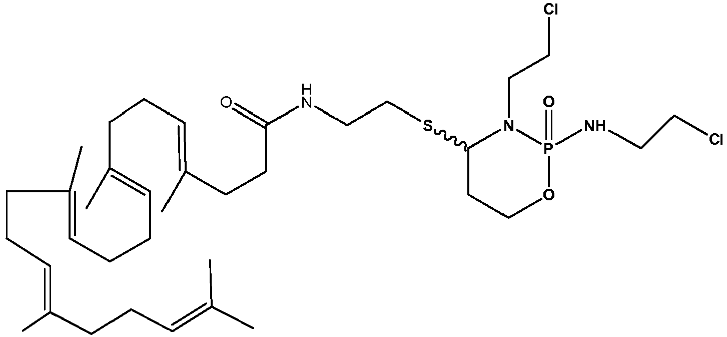

Protocole : A partir du 4-méthoxy-ifosfamide, est ajouté dans des conditions similaires aux produits précédents (-78°C, CH₂Cl₂, BF₃, EtO₂, 30 min) 1 équivalent de cystéamidosqualènique acide pour former le composé attendu avec un rendement de 60%.

Les produits obtenus sont purifiés par chromatographie.
Organoleptique : huile légèrement jaune.
*Formule brute :* C₃₆H₆₄Cl₂N₃O₃PS
*IR (film)* ; ν (cm⁻¹) : 2956 (C-H), 2352, 1746, (C=0), 1638 (C=C), 1432 (P-N), 1258 (P=O), 1063-1109 (P-O-C)

*RMN* dans CDCl₃.
*¹³**C*** δ (ppm) : **33,4** (C₅) ; **35,6** (C₁₂) ; **36,9** (C₁₃) ; **42,6** (C₉) ; **44,5** (C₇) ; **47,3** (C₈) ; **50,0** (C₁₀) ; **63,5** (C₆) ; **67,8** (C₁₁) ; **89,5** (C₄) ; **125,9** (C_{ethylen.}) ; **132,0** (C_{quat}).
*¹**H*** δ (ppm) : **0,95** (sl, 1H, NH) ; **1,30** (m, 2H, 2H₁₃) ; **1,55** (s, 18H) ; **1,95** (m, 20H) ; **3,20** (m, 2H, 2H₉) ; **3,35** (m, 2H, 2H₇) ; **3,55** (m, 2H, 2H₈) ; **3,65** (m, 2H, 2H₁₀) ; **4,25** (m, 1H, H_{6Eq.}) ; **4,50** (m, 1H, H_{6Ax.}) ; **4,65** (m, 1H , H₄) ; **5,05** (m, 5H , H_{ethyleniques}), **7,35** (sl, 1H, NH).

Remarque : l'acide cystéamido-squalénique et le tris-nor squalènol peuvent être obtenus à partir du squalène aldéhyde par des méthodes de synthèse classique. La synthèse de dérivés aldéhyde du squalène est décrite dans Ceruti et al., J. Chem. Soc, Perkin Trans. 1, 2002, 1477-1486 (voir schéma 2 p. 1479).

### Exemple 2 : Evaluation biologique des composés 4-thio SQ-IFO et 4-SQ-IFO

### I. Evaluation de la cytotoxicité in vitro

### I.1. Matériel et Méthodes:

### Culture cellulaire et conditions de culture

La cytotoxicité des nanoparticules de SQ-IFO et de SQ-thio-IFO a été étudiée sur plusieurs lignées:
▪ A549 : adénocarcinome humain alvéolaire des cellules épithéliales basales.
▪ MCF-7 : carcinoma mammaire humain
▪ MCF-7 MDR : carcinoma mammaire humain résistant
▪ B16F10 : melanoma de souris
▪ KB 3.1 : carcinome épidermoide humain
▪ M109 : cellules tumorales pulomonaire de souris
▪ MiaPaCa-2 : carcinoma pancréatique humain
▪ UW-479 : gliome pédiatrique humain
▪ IGR OV1 : cancer de l'ovaire humain
▪ SK-N-MC: neuroectodermal, reclassé en Ewing (expression de l'oncogène EWS/Flip-1)

Les lignées sont maintenues dans du milieu de culture DMEM ou RPMI supplémenté avec 10% de sérum de veau foetal et 1% d'antibiotiques (100 U/mL de pénicilline et 100 µg/mL de streptomycine) dans un incubateur à 37 °C avec 5% de dioxyde de carbone en atmosphère humide.

Les cellules sont ensemencées en plaques 96 puits (TPP). L'ensemencement a été optimisé pour chaque lignée pour un temps d'incubation de 72 heures.

Les cellules A549, MCF-7, B16F10, M109, MiaPaCa-2 sont ensemencées à 5.10³ cellules/puits. KB 1.3 est ensemencée à 2.10³ cellules/puits. IGR-OV1 sont ensemencées à 10⁴ cellules/puits et UW479 et SK-N-MC à 5.10⁴ cellules/puits.

### Préparation des suspensions de nanoparticules

Les composés SQ-IFO et SQ-thio-IFO ont été synthétisés selon le protocole précédemment décrit. Pour chaque composé, une suspension aqueuse de nanoparticules a été préparée selon la technique de nanoprécipitation décrite dans Fessi, Int. J. Pharm, 19889, 55, R1-R4. Les nanoparticules obtenues sont sphériques et présentent une taille moyenne de 182 nm.

Pour chaque composé, différentes concentrations comprises entre 0,1 et 100 µM ont été testées par dilution successive dans du milieu de culture d'une solution mère à 2mg/ml pour le SQ-IFO et d'une solution mère à 10 mg/ml pour le SQ-thio-IFO.

### Test de viabilité cellulaire

Après 72 heures d'incubation, la viabilité cellulaire est déterminée par observation de la réduction du réactif MTS en formazan (Kit Cell titer 96 Aquerus one solution, Promega). 20 µL d'une solution à 5 mg/mL de MTS dans du PBS sont ajoutés par puits. Le temps d'incubation du MTS est optimisé pour chaque lignée, puis la lecture de l'absorbance à 490 nm est ensuite réalisée grâce à un lecteur de plaque (EL808, Biotek).

Les résultats sont exprimés en pourcentage des cellules non traitées. Les données ont traité avec le logiciel Prism 4 (Graph Pad Software, San Diego). Les IC50 retrouvées sont ainsi calculées pour chaque lignée. L'ifosfamide et le squalénol ont été utilisés à titre de contrôle.

### I.2. Résultats

Le tableau 5 ci-dessous présente, pour chaque composé testé, l'IC50 obtenue pour chaque lignée cellulaire.

Il apparaît clairement que les composés IFO-SQ et IFO-thio-SQ présentent une cytotoxicité *in vitro* élevée : ces composés sont ainsi capables de libérer la moutarde alkylante sans activation préalable par les cytochromes P450.

De manière remarquable, on note un profil d'activité différent pour IFO-thio-SQ et IFO-SQ en fonction de la lignée cellulaire cancéreuse. IFO-thio-SQ présente une activité cytotoxique élevée vis-à-vis des lignées cellulaires M109, SK-N-MC (Ewing), UW 479 (Gliome) et IGR-OV1 (Ovaire). Une telle activité n'est pas observée pour le composé IFO-SQ.

Le composé squalénol et l'IFO n'exercent pas d'effet cytotoxique notable aux concentrations testées.

**Tableau 5 : IC50 des composés IFO, SQ-IFO et SQ-thio-IFO obtenue pour chaque lignée cellulaire testée.**

| IC50 (µM) | Ifosfamide | Ifosfamide SQ | Ifosfamide thio SQ |
|---|---|---|---|
| A549 | > 100 | 32,5 | 5,3 |
| MCF-7 | > 100 | 43,6 | 10,9 |
| MCF-7 MDR | > 100 | 78,8 | 80 |
| B16F10 | > 100 | 73 | 16,9 |
| KB 3.1 | > 100 | 50 | 2,96 |
| M109 | > 100 | > 100 | 9,2 |
| MiaPaCa-2 | > 100 | 32,5 | 4,4 |
| SK-N-MC (Ewing) | > 100 | > 100 | 19 |
| UW 479 (Gliome) | > 100 | > 100 | 65 |
| IGR-OV1 (Ovaire) | > 100 | > 100 | 81 |

### II. Evaluation de l'efficacité in vivo

L'efficacité cytotoxique in vivo a été évaluée en premier lieu sur un modèle de rhabdomyosarcome humain xénogreffé à la souris nude.Le modèle utilisé est le modèle RD de rhabdomyosarcome pédiatrique humain. Les cellules ont été tout d'abord amplifiées en culture, puis comptées. 10.10⁶ cellules ont été injectées en sous-cutanée au niveau des deux flancs de chaque souris. Après prise tumorale, et atteinte des volumes tumoraux supérieurs à 80 mm³, les souris ont été traitées avec le SQ-IFO ou avec un placebo. Les résultats préliminaires de cette étude montrent une diminution importante des volumes tumoraux chez les souris traitées par le SQ-IFO comparativement aux souris traitées avec le placebo. Des résultats analogues sont attendus pour le composé SQ-thio-IFO.

## Revendications

1. Composé de formule (I) 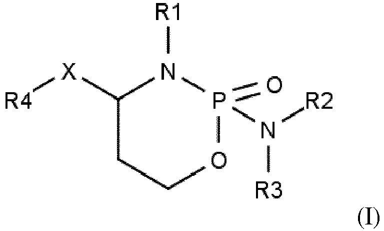 dans laquelle
- X est O ou S ;
- R2 est H ;
- R1 et R3 sont indépendamment -(CH₂)₂-Cl ou -CH(CH₃)-CH₂-Cl; et,
- R4 est de formule R5(Y)ₐ dans laquelle :
- Y représente un espaceur, de préférence choisi parmi le groupe constitué par -(CH₂)ₘ-, -CONH(CH₂)ₘ-, -NHCO(CH₂)ₘ-, -COO(CH₂)ₘ- et -OCO(CH₂)ₘ- avec m est un entier allant de 1 à 10 ;
- a est 1 ; et
- R₅ est un groupe hydrocarboné de 3 à 30 atomes de carbone, linéaire ou ramifié,
ou un sel pharmaceutiquement acceptable de celui-ci.

2. Composé selon la revendication 1, dans lequel R1 et R3 sont tous deux soit -(CH₂)₂Cl ou soit - CH(CH₃)CH₂Cl.

3. Composé selon la revendication 1 ou 2, dans lequel R5 est un groupe hydrocarboné de 3 à 30 atomes de carbone, saturé, linéaire ou ramifié..

4. Composé selon la revendication 1 ou 2, dans lequel R5 est un groupe hydrocarboné de 3 à 30 atomes de carbone, insaturé, linéaire ou ramifié.

5. Composé selon la revendication 1 ou 2, dans lequel R5 comprend de 1 à 6 ramifications, lesdites ramifications étant des groupes méthyles, et/ou de 1 à 4 insaturations.

6. Composé selon la revendication 1 ou 2, dans lequel R5 comprend un ou plusieurs motifs dérivés de l'isoprène.

7. Composé selon la revendication 1 ou 2, dans lequel R5 est choisi parmi le groupe constitué par :
(a) (CH₃)₂C=CH-CH₂-CH₂-[C(CH₃)=CH-CH₂-CH₂]ₘ- avec m un entier allant de 0 à 5, et
(b) (CH₃)₂C=CH-CH₂-CH₂-[C(CH₃)=CH-CH₂-CH₂]ₚ-[CH=C(CH)₃-CH₂-CH₂]_{q}- avec p est un entier allant de 1 à 5 et q est un entier allant de 1 à 5.

8. Composé selon l'une quelconque des revendications 1 ou 2, dans lequel R4 est un radical squalènoyle.

9. Composé selon la revendication 1, dans lequel R2 est H et R1 et R3 sont -(CH₂)₂-Cl et R4 est un radical squalènoyle, X pouvant être O ou S.

10. Composé selon la revendication 1, ledit composé étant choisi parmi : 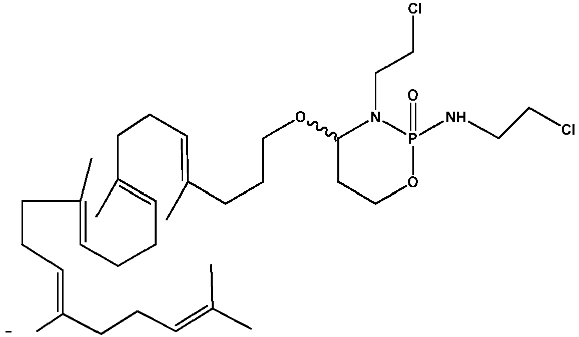 ou

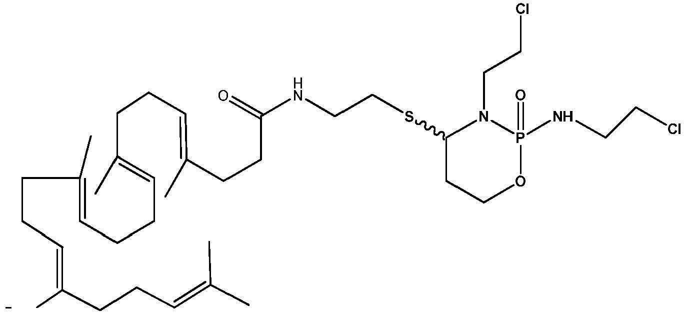

11. Nanoparticule formée par un composé de formule (I) tel que définie dans l'une quelconque des revendications 1 à 10.

12. Nanoparticule selon la revendication 11, dans laquelle le composé de formule (I) est tel que :
- X est O ou S;
- R2 est H;
- R1 et R3 sont indépendamment -CH(CH₃)-CH₂-Cl ou -(CH₂)₂-Cl ; et,
- R4 est un radical squalènoyle ;
ou un sel pharmaceutiquement acceptable de celui-ci.

13. Nanoparticule selon la revendication 11, dans laquelle le composé de formule (I) est tel que défini dans la revendication 10.

14. Composition pharmaceutique comprenant Un composé de formule (I) tel que défini dans l'une quelconque des revendications 1 à 10 ou comprenant une nanoparticule selon l'une quelconque des revendications 11 à 13.

15. Composition pharmaceutique selon la revendication 14 pour une utilisation dans le traitement du cancer ou comme immunosuppresseur.

16. Méthode de préparation d'un composé de formule (I) : 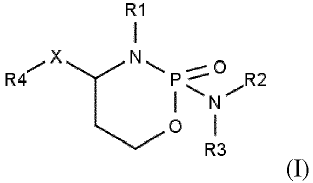 telle que définie dans l'une quelconque des revendications 1 à 10, ladite méthode comprenant :
- la fourniture d'un composé de formule (II) 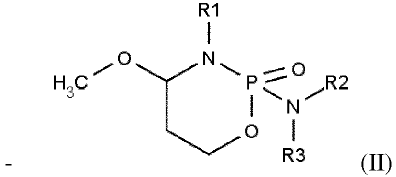
- dans laquelle R1, R2 et R3 sont tels que définis pour la formule (I); et,
- la réaction du composé de formule (II) avec un alcool ou un thiol de formule (III) R4-XH, dans laquelle X et R4 sont tels que définis dans la formule (I), en présence d'un acide de Lewis, ledit acide de Lewis étant BF₃.OEt₂.

## Patentansprüche

1. Verbindung der Formel (I) 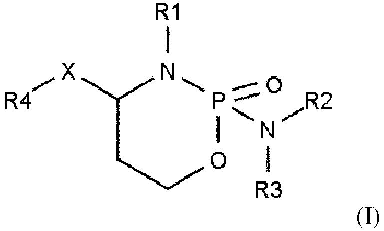 in der
- XOoderSist;
- R2 H ist;
- R1 und R3 unabhängig voneinander -(CH₂)₂-Cl oder -CH(CH₃)-CH₂-Cl sind; und
- R4 die Formel R5(Y)ₐ besitzt, in der:
- Y für einen Spacer steht, vorzugsweise ausgewählt aus der Gruppe, bestehend aus -(CH₂)ₘ-, -CONH(CH₂)ₘ-, -NHCO(CH₂)ₘ-, -COO(CH₂)ₘ- und -OCO(CH₂)ₘ-, wobei m eine ganze Zahl von 1 bis 10 ist;
- a 1 ist; und
- R5 eine lineare oder verzweigte Kohlenwasserstoffgruppe mit 3 bis 30 Kohlenstoffatomen ist,
oder ein pharmazeutisch verträgliches Salz davon.

2. Verbindung gemäß Anspruch 1, wobei R1 und R3 entweder beide -(CH₂)₂Cl oder beide - CH(CH₃)CH₂Cl sind.

3. Verbindung gemäß Anspruch 1 oder 2, wobei R5 eine lineare oder verzweigte gesättigte Kohlenwasserstoffgruppe mit 3 bis 30 Kohlenstoffatomen ist.

4. Verbindung gemäß Anspruch 1 oder 2, wobei R5 eine lineare oder verzweigte ungesättigte Kohlenwasserstoffgruppe mit 3 bis 30 Kohlenstoffatomen ist.

5. Verbindung gemäß Anspruch 1 oder 2, wobei R5 1 bis 6 Verzweigungen, wobei besagte Verzweigungen Methylgruppen sind, und/oder 1 bis 4 ungesättigte Bindungen umfasst.

6. Verbindung gemäß Anspruch 1 oder 2, wobei R5 eine oder mehrere Isopren-Einheiten umfasst.

7. Verbindung gemäß Anspruch 1 oder 2, wobei R5 aus der Gruppe ausgewählt ist, bestehend aus:
(a) (CH₃)₂C=CH-CH₂-CH₂-[C(CH₃)=CH-CH₂-CH₂]ₘ-, wobei m eine ganze Zahl von 0 bis 5 ist, und
(b) (CH₃)₂C=CH-CH₂-CH₂-[C(CH₃)=CH-CH₂-CH₂]ₚ-[CH=C(CH)₃-CH₂-CH₂]_{q}-, wobei p eine ganze Zahl von 1 bis 5 ist und q eine ganze Zahl von 1 bis 5 ist.

8. Verbindung gemäß einem der Ansprüche 1 oder 2, wobei R4 eine Squalenoyl-Gruppe ist.

9. Verbindung gemäß Anspruch 1, wobei R2 H ist und R1 und R3 -(CH₂)₂-Cl sind und R4 eine Squalenoyl-Gruppe ist, wobei X O oder S sein kann.

10. Verbindung gemäß Anspruch 1, wobei besagte Verbindung ausgewählt ist aus: 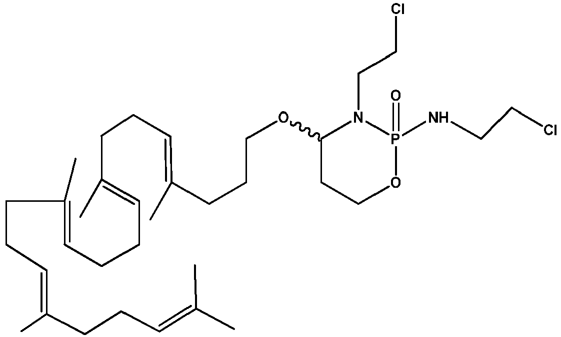 oder

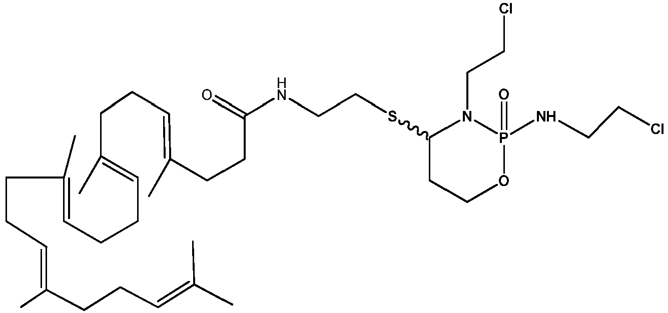

11. Nanoteilchen, das von einer wie in einem der Ansprüche 1 bis 10 definierten Verbindung der Formel (I) gebildet wird.

12. Nanoteilchen gemäß Anspruch 11, wobei die Verbindung der Formel (I) wie beispielsweise ist:
- XOoderSist;
- R2 H ist;
- R1 und R3 unabhängig voneinander -CH(CH₃)-CH₂-Cl oder -(CH₂)₂-Cl sind; und
- R4 eine Squalenoyl-Gruppe ist;
oder ein pharmazeutisch verträgliches Salz davon.

13. Nanoteilchen gemäß Anspruch 11, wobei die Verbindung der Formel (I) wie in Anspruch 10 definiert ist.

14. Pharmazeutische Zusammensetzung, umfassend eine Verbindung der Formel (I) wie in einem der Ansprüche 1 bis 10 definiert oder umfassend ein Nanoteilchen gemäß einem der Ansprüche 11 bis 13.

15. Pharmazeutische Zusammensetzung gemäß Anspruch 14 zur Verwendung in der Behandlung von Krebs oder als Immunsuppressor.

16. Verfahren zur Herstellung einer Verbindung der Formel (I): 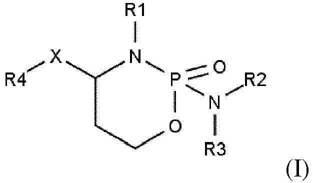 wie in einem der Ansprüche 1 bis 10 definiert,
wobei besagtes Verfahren umfasst:
- die Bereitstellung einer Verbindung der Formel (II) 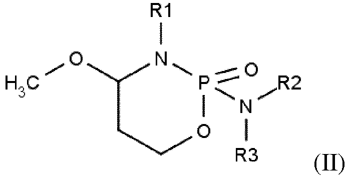
- in der R1, R2 und R3 wie für die Formel (I) definiert sind; und
- die Reaktion der Verbindung der Formel (II) mit einem Alkohol oder einem Thiol der Formel (III) R4-XH, in der X und R4 wie in Formel (I) definiert sind, in Gegenwart einer Lewis-Säure, wobei besagte Lewis-Säure BF₃·OEt₂ ist.

## Claims

1. A compound of formula (I) 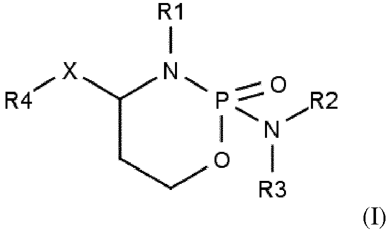 wherein
- XisOorS;
- R2 is H;
- R1 and R3 are independently -(CH₂)₂-Cl or -CH(CH₃)-CH₂-Cl; and,
- R4 is of formula R5(Y)ₐ wherein:
- Y is a spacer, preferably selected from the group consisting of -(CH₂)ₘ-, -CONH(CH₂)ₘ-, -NHCO(CH₂)ₘ-, -COO(CH₂)ₘ- et -OCO(CH₂)ₘ- where m is an integer ranging from 1 to 10;
- a is 1; and
- R5 is a hydrocarbon group of 3 to 30 carbon atoms, linear or branched;
or a pharmaceutically acceptable salt thereof.

2. The compound according to claim 1, wherein R1 and R3 are both either -(CH₂)₂-Cl or -CH(CH₃)-CH₂-Cl.

3. The compound according to claim 1 or 2, wherein R5 is a hydrocarbon group of 3 to 30 carbon atoms, saturated, linear or branched.

4. The compound according to claim 1 or 2, wherein R5 is a hydrocarbon group of 3 to 30 carbon atoms, unsaturated, linear or branched.

5. The compound according to claim 1 or 2, wherein R5 comprises from 1 to 6 branching, said branching being methyl groups, and/or from 1 to 4 unsaturations.

6. The compound according to claim 1 or 2, wherein R5 comprises one or several moieties derived from isoprene.

7. The compound according to claim 1 or 2, wherein R5 is selected from the group consisting of:
(a) (CH₃)₂C=CH-CH₂-CH₂-[C(CH₃)=CH-CH₂-CH₂]ₘ- with m an integer ranging from 0 to 5, and
(b) (CH₃)₂C=CH-CH₂-CH₂-[C(CH₃)=CH-CH₂-CH₂]ₚ-[CH=C(CH)₃-CH₂-CH₂]_{q}- with p an integer ranging from 1 to 5 and q an integer ranging from 1 to 5.

8. The Compound according to any one of claims 1 or 2, wherein R4 is a squalenoyl group.

9. The compound according to claim 1, wherein R2 is H and R1 and R3 are -(CH₂)₂-Cl and R4 is a squalenoyl group, X may be O or S.

10. The compound according to claim 1, said compound being selected from: 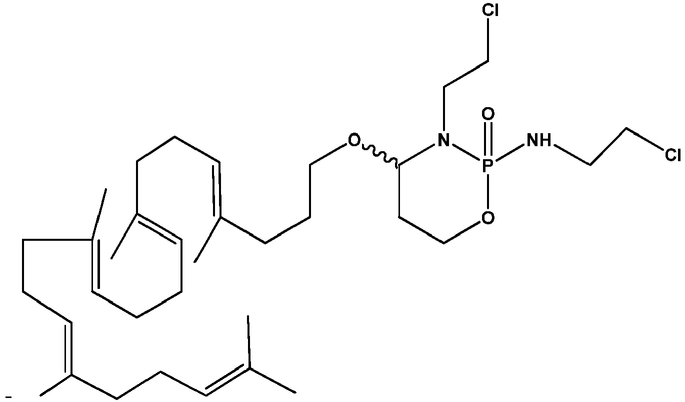 or

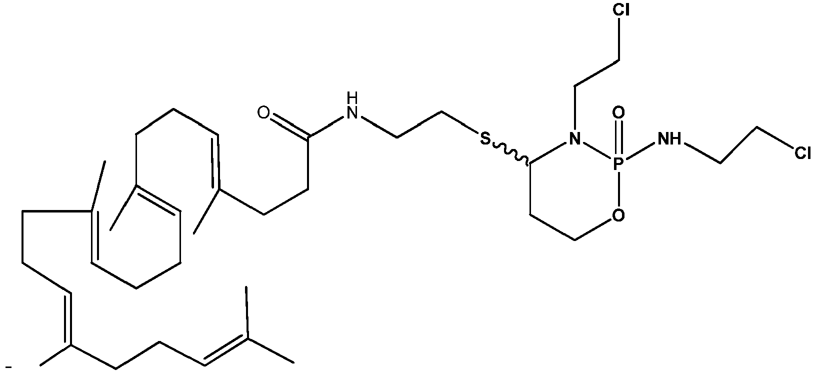

11. Nanoparticle formed by the compound of formula (I) as defined in anyone of claims 1 to 10.

12. The nanoparticle according to claim 11, wherein the compound of formula (I) is such as:
- XisOorS;
- R2 is H;
- R1 and R3 are independently -CH(CH₃)-CH₂-Cl or -(CH₂)₂-Cl; and,
- R4 is a squalenoyl group;
or a pharmaceutically acceptable salt thereof.

13. The nanoparticle according to claim 11, wherein the compound of formula (I) is as defined in claim 10.

14. A pharmaceutical composition comprising a compound of formula (I) as defined in any one of claims 1 to 10 or comprising a nanoparticle according to anyone of claims 11 to 13.

15. The pharmaceutical composition according to claim 14 for use in the treatment of cancer or as an immunosuppressant.

16. A method for preparing a compound of formula (I): 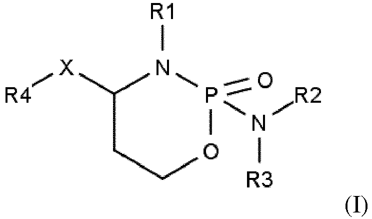 as defined in anyone of claims 1 to 10,
said method comprising:
- providing a compound of formula (II) 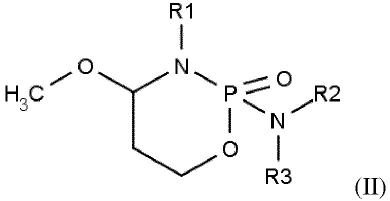
- wherein R1, R2 and R3 are as defined in formula (I); and,
- reacting the compound of formula (II) with an alcohol or a thiol of formula (III) R4-XH, wherein X and R4 are as defined in formula (I), in the presence of a Lewis acid, said Lewis acid being BF₃.OEt₂.
